(19) Europäisches Patentamt
European Patent Office
Office européen des brevets

(11) **EP 4 438 091 B1**

(12) **EUROPEAN PATENT SPECIFICATION**

(45) Date of publication and mention
of the grant of the patent:
**01.04.2026 Bulletin 2026/14**

(21) Application number: **24185955.2**

(22) Date of filing: **21.02.2020**

(51) International Patent Classification (IPC):
***A61M 16/00*** *(2006.01)*

(52) Cooperative Patent Classification (CPC):
(C-Sets available)
**A61M 16/024;** A61M 16/0069; A61M 16/0672;
A61M 16/1095; A61M 16/161; A61M 16/209;
A61M 2016/0021; A61M 2016/0027;
A61M 2016/0036; A61M 2205/3317;
A61M 2205/3334; A61M 2205/3365;
A61M 2205/3368; A61M 2205/3375;
A61M 2205/3561; (Cont.)

(54) **ADJUSTABLE EXPIRATORY RELIEF IN RESPIRATORY THERAPY**

EINSTELLBARE EXSPIRATORISCHE ENTLASTUNG IN DER ATEMTHERAPIE

LIBÉRATION EXPIRATOIRE RÉGLABLE EN THÉRAPIE RESPIRATOIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.02.2019 US 201962809099 P**

(43) Date of publication of application:
**02.10.2024 Bulletin 2024/40**

(62) Document number(s) of the earlier application(s) in
accordance with Art. 76 EPC:
**20759717.0 / 3 927 405**

(73) Proprietor: **Fisher & Paykel Healthcare Limited
East Tamaki, Auckland 2013 (NZ)**

(72) Inventor: **BURGESS, Russel William
2013 Auckland (NZ)**

(74) Representative: **Haseltine Lake Kempner LLP
One Portwall Square
Portwall Lane
Bristol BS1 6BH (GB)**

(56) References cited:
**WO-A1-2017/200394    US-A1- 2016 015 918**

(52) Cooperative Patent Classification (CPC): (Cont.)
A61M 2205/3569; A61M 2205/3584;
A61M 2205/3592; A61M 2205/505; A61M 2230/06;
A61M 2230/205; A61M 2230/42

C-Sets
A61M 2230/06, A61M 2230/005;
A61M 2230/205, A61M 2230/005;
A61M 2230/42, A61M 2230/005

**Description**

FIELD OF THE DISCLOSURE

The present disclosure relates to systems for flow therapy that deliver gas to patients using a flow therapy apparatus.

BACKGROUND

Breathing assistance apparatuses are used in various environments such as hospital, medical facility, residential care, or home environments to deliver a flow of gas to users or patients. A breathing assistance apparatus, or a flow therapy apparatus, may include a humidification apparatus to deliver heated and humidified gases. The apparatus may optionally include a valve used to deliver oxygen with the flow of gas. A flow therapy apparatus may allow adjustment and control over characteristics of the gas flow, including flow rate, temperature, gas concentration, humidity, pressure, etc. Sensors, such as heated temperature sensing elements and/or thermistors, are used to measure these properties of the gases.

WO2017/200394 A1 discloses such a flow therapy apparatus.

SUMMARY

According to the present invention there is provided a respiratory system according to claim 1. Further embodiments are object of the dependent claims.

The flow of respiratory gases in a flow therapy apparatus can be adjusted based upon a detected breath cycle of the patient. The flow of gases can be adjusted such that the flow delivered during expiration is lower than the flow delivered during inspiration. The adjustment may improve patient comfort. The system can synchronize the flow rate with the detected breath cycle of the patient. The amplitude of the flow rate variation can be based partially on a value selected by the user. The flow rate can be adjusted by controlling the motor speed using a positive feedback system. The adjustments to the flow rate can be limited by the controller preventing the flow rate from crossing a minimum and/or maximum threshold, which can be varied. The adjustment can be made to the motor speed, that is, the positive feedback, which can cause the adjustment to the flow rate.

When the patient is exhaling, the threshold(s) or limit(s) can ensure that the apparatus is maintaining at least a minimum flow rate to the patient at all times while also providing expiratory flow relief. Maintaining the threshold can ensure that the flow rate does not fall outside a clinically relevant or effective value and/or a safety limit for a respiratory therapy, particularly for a high flow therapy. A user of the apparatus, such as a clinician and/or the patient, can also adjust the value of variation in the flow rate. The user-adjustment can allow the user to select a value of flow relief that the patient finds most comfortable.

In a configuration, a respiratory system for delivering a respiratory therapy to a patient, the system configured to adjust a flow rate of gases delivered to the patient according to patient inspiration and expiration, can comprise a flow generator configured to generate the flow rate of gases; a controller in electrical communication with one or more sensors and configured to: determine a cycle of the patient inspiration and expiration based on information received from the one or more sensors; and adjust the flow rate of gases based in part on the cycle of the patient inspiration and expiration, wherein the adjusting can be attenuated by a parameter determined based in part on a maximum and/or minimum flow rate measured by the one or more sensors during the cycle of the patient inspiration and expiration.

In a configuration, the flow generator can comprise a motor.

In a configuration, the flow rate can be adjusted by outputting a motor control signal.

In a configuration, the motor can comprise a brushless DC motor.

In a configuration, the cycle of the patient inspiration and expiration can be determined based on a first input and a second inputs received by the controller, the first and second inputs relating to a gases flow characteristic or performance of a component of the system.

In a configuration, the first input can correspond to the flow rate from the one or more sensors.

In a configuration, the second input can correspond to a pressure of a gases flow from the one or more sensors. In a configuration, the second input can correspond to a speed of the motor.

In a configuration, the speed of the motor can be determined based at least in part upon one or more motor parameters.

In a configuration, the maximum and/or minimum flow rate measured by the one or more sensors can be an unbounded response to the patient inspiration and expiration.

In a configuration, the adjusting can be further attenuated by comparing the maximum and/or minimum flow rate measured by the one or more sensors with a maximum and/or minimum threshold.

In a configuration, the maximum and/or minimum flow rate measured by the one or more sensors can be gradually bounded by a negative feedback term.

In a configuration, the parameter can comprise the negative feedback term that can be gradually increased in response to the measured maximum and/or minimum flow rates until the measured maximum and/or minimum flow rate no longer exceeds the maximum and/or minimum threshold.

In a configuration, the maximum and/or minimum threshold can be set by a user.

In a configuration, the system can comprises a user interface configured to receive a user input to adjust the maximum and/or minimum threshold.

In a configuration, the maximum and/or minimum threshold can be a clinically relevant limit of the respiratory therapy.

In a configuration, the minimum threshold can be between 0-5 lpm. In a configuration, the minimum threshold can be between 10 lpm. In a configuration, the minimum threshold can be between 10-15 lpm. In a configuration, the minimum threshold can be between 15-20 lpm. In a configuration, the minimum threshold can be between 20-25 lpm. In a configuration, the minimum threshold can be between 25-30 lpm. In a configuration, the minimum threshold can be between 30-35 lpm. In a configuration, the minimum threshold can be between 35-40 lpm. In a configuration, the minimum threshold can be between 40-45 lpm. In a configuration, the minimum threshold can be between 45-50 lpm. In a configuration, the minimum threshold can be between 50-55 lpm. In a configuration, the minimum threshold can be between 55-60 lpm.

In a configuration, the maximum threshold can be between 20-25 lpm. In a configuration, the maximum threshold can be between 25-30 lpm. In a configuration, the maximum threshold can be between 30-35 lpm. In a configuration, the maximum threshold can be between 35-40 lpm. In a configuration, the maximum threshold can be between 40-45 lpm. In a configuration, the maximum threshold can be between 45-50 lpm. In a configuration, the maximum threshold can be between 50-55 lpm. In a configuration, the maximum threshold can be between 55-60 lpm. In a configuration, the maximum threshold can be between 60-65 lpm. In a configuration, the maximum threshold can be between 65-70 lpm. In a configuration, the maximum threshold can be between 70-75 lpm. In a configuration, the maximum threshold can be between 75-80 lpm. In a configuration, the maximum threshold can be between 80-85 lpm. In a configuration, the maximum threshold can be between 85-90 lpm. In a configuration, the maximum threshold can be between 90-95 lpm. In a configuration, the maximum threshold can be between 95-100 lpm.

In a configuration, the maximum and/or minimum threshold can be a safety limit of the respiratory therapy.

In a configuration, the maximum and/or minimum threshold can depend on a selected flow rate.

In a configuration, the maximum and/or minimum threshold can be a predetermined percentage or value above and below the selected flow rate.

In a configuration, the maximum and/or minimum threshold can depend on a selected flow rate and a selected breath synchronization setting.

In a configuration, the system can comprise a user interface configured to receive a user input to adjust the breath synchronization setting.

In a configuration, the breath synchronization setting can comprise a range of numbers. In a configuration, the breath synchronization setting can comprise a plurality of categories including at least low and high settings.

In a configuration, the maximum and/or minimum threshold can be a predetermined percentage or value above and below the selected flow rate, the percentage or value varying based on the selected breath synchronization setting.

In a configuration, the adjusting can be performed iteratively.

In a configuration, the adjusting can comprise an increase in the flow rate when the patient is inspiring.

In a configuration, the adjusting can comprise a decrease in the flow rate when the patient is expiring.

In a configuration, the one or more sensors can comprise an ultrasonic transducer assembly.

In a configuration, the one or more sensors can comprise a heated temperature sensing element.

In a configuration, the system can be a high flow respiratory system.

In a configuration, the system can comprise a non-sealed patient interface.

In a configuration, the non-sealed patient interface can comprise a non-sealed nasal cannula.

In a configuration, a respiratory system for delivering a respiratory therapy to a patient, the system configured to adjust a flow rate of gases delivered to the patient according to patient inspiration and expiration, can comprise a flow generator configured to generate the flow rate of gases; and a controller in electrical communication with one or more sensors and configured to: determine a cycle of the patient inspiration and expiration based on information received from the one or more sensors; and adjust the flow rate based in part on the cycle of the patient inspiration and expiration, wherein the adjusting can be attenuated by comparing a maximum and/or minimum flow rate measured by the one or more sensors with a maximum and/or minimum threshold.

In a configuration, the flow generator can comprise a motor.

In a configuration, the flow rate can be adjusted by outputting a motor control signal.

In a configuration, the motor can comprise a brushless DC motor.

In a configuration, the cycle of the patient inspiration and expiration can be determined based on a first input and a second inputs received by the controller, the first and second inputs relating to a gases flow characteristic or performance of a component of the system.

In a configuration, the first input can correspond to the flow rate from the one or more sensors.

In a configuration, the second input can correspond a pressure of a gases flow from the one or more sensors. In a configuration, the second input can correspond to a speed of the motor.

In a configuration, the speed of the motor can be determined based at least in part upon one or more motor parameters.

In a configuration, the maximum and/or minimum threshold can be set by a user.

In a configuration, the system can comprises a user interface configured to receive a user input to adjust the maximum and/or minimum threshold.

In a configuration, the maximum and/or minimum threshold can be a clinically relevant limit of the respiratory therapy.

In a configuration, the minimum threshold can be between 0-5 lpm. In a configuration, the minimum threshold can be between 10 lpm. In a configuration, the minimum threshold can be between 10-15 lpm. In a configuration, the minimum threshold can be between 15-20 lpm. In a configuration, the minimum threshold can be between 20-25 lpm. In a configuration, the minimum threshold can be between 25-30 lpm. In a configuration, the minimum threshold can be between 30-35 lpm. In a configuration, the minimum threshold can be between 35-40 lpm. In a configuration, the minimum threshold can be between 40-45 lpm. In a configuration, the minimum threshold can be between 45-50 lpm. In a configuration, the minimum threshold can be between 50-55 lpm. In a configuration, the minimum threshold can be between 55-60 lpm.

In a configuration, the maximum threshold can be between 20-25 lpm. In a configuration, the maximum threshold can be between 25-30 lpm. In a configuration, the maximum threshold can be between 30-35 lpm. In a configuration, the maximum threshold can be between 35-40 lpm. In a configuration, the maximum threshold can be between 40-45 lpm. In a configuration, the maximum threshold can be between 45-50 lpm. In a configuration, the maximum threshold can be between 50-55 lpm. In a configuration, the maximum threshold can be between 55-60 lpm. In a configuration, the maximum threshold can be between 60-65 lpm. In a configuration, the maximum threshold can be between 65-70 lpm. In a configuration, the maximum threshold can be between 70-75 lpm. In a configuration, the maximum threshold can be between 75-80 lpm. In a configuration, the maximum threshold can be between 80-85 lpm. In a configuration, the maximum threshold can be between 85-90 lpm. In a configuration, the maximum threshold can be between 90-95 lpm. In a configuration, the maximum threshold can be between 95-100 lpm.

In a configuration, the maximum and/or minimum threshold can be a safety limit of the respiratory therapy.

In a configuration, the maximum and/or minimum threshold can depend on a selected flow rate.

In a configuration, the maximum and/or minimum threshold can be a predetermined percentage or value above and below the selected flow rate.

In a configuration, the maximum and/or minimum threshold can depend on a selected flow rate and a selected breath synchronization setting.

In a configuration, the system can comprise a user interface configured to receive a user input to adjust the breath synchronization setting.

In a configuration, the breath synchronization setting can comprise a range of numbers. In a configuration, the breath synchronization setting can comprise a plurality of categories including at least low and high settings.

In a configuration, the maximum and/or minimum threshold can be a predetermined percentage or value above and below the selected flow rate, the percentage or value varying based on the selected breath synchronization setting.

In a configuration, the adjusting can be performed iteratively.

In a configuration, each adjusting can be bound by a parameter determined based in part on a maximum and/or minimum flow rate measured by the one or more sensors.

In a configuration, the maximum and/or minimum flow rate measured by the one or more sensors can be an unbounded response to the patient inspiration and expiration.

In a configuration, the maximum and/or minimum flow rate measured by the one or more sensors can be gradually bounded by a negative feedback term.

In a configuration, the parameter can comprise the negative feedback term that can be gradually increased in response to the measured maximum and/or minimum flow rates until the measured maximum and/or minimum flow rate no longer exceeds the maximum and/or minimum threshold.

In a configuration, the adjusting can comprise an increase in the flow rate when the patient is inspiring.

In a configuration, the adjusting can comprise a decrease in the flow rate when the patient is expiring.

In a configuration, the one or more sensors can comprise an ultrasonic transducer assembly.

In a configuration, the one or more sensors can comprise a heated temperature sensing element.

In a configuration, the system can be a high flow respiratory system.

In a configuration, the system can comprise a non-sealed patient interface.

In a configuration, the non-sealed patient interface can comprise a non-sealed nasal cannula.

In a configuration, a method of adjusting a flow rate of gases delivered to a patient according to patient inspiration and expiration using a respiratory system comprising a flow generator configured to generate the flow rate of gases can comprise using a controller of the respiratory system: determining a cycle of inspiration and expiration of the patient based on information received from one or more sensors of the respiratory system; and adjusting the flow rate based in part on the cycle of inspiration and expiration, wherein the adjusting can be attenuated by a parameter determined based in part on a maximum and/or minimum flow rate measured by the one or more sensors during the cycle of inspiration and expiration.

In a configuration, the flow generator can comprise a motor.

In a configuration, the flow rate can be adjusted by outputting a motor control signal.

In a configuration, the motor can comprise a brushless DC motor.

In a configuration, the determining can be based on a first input and a second inputs received by the controller, the first and second inputs relating to a gases flow characteristic or performance of a component of the system.

In a configuration, the first input can correspond to the flow rate from the one or more sensors.

In a configuration, the second input can correspond to a pressure of a gases flow from the one or more sensors. In a configuration, the second input can correspond to a speed of the motor.

In a configuration, the method can further comprise determining the speed of the motor based at least in part upon one or more motor parameters.

In a configuration, the maximum and/or minimum flow rate measured by the one or more sensors can be an unbounded response to the patient inspiration and expiration.

In a configuration, the adjusting can be further attenuated by comparing the maximum and/or minimum flow rate measured by the one or more sensors with a maximum and/or minimum threshold.

In a configuration, the maximum and/or minimum flow rate measured by the one or more sensors can be gradually bounded by a negative feedback term.

In a configuration, the parameter can comprise the negative feedback term that can be gradually increased in response to the measured maximum and/or minimum flow rates until the measured maximum and/or minimum flow rate no longer exceeds the maximum and/or minimum threshold.

In a configuration, the maximum and/or minimum threshold can be set by a user.

In a configuration, the system can comprises a user interface configured to receive a user input to adjust the maximum and/or minimum threshold.

In a configuration, the maximum and/or minimum threshold can be a clinically relevant limit of the respiratory therapy.

In a configuration, the minimum threshold can be between 0-5 lpm. In a configuration, the minimum threshold can be between 10 lpm. In a configuration, the minimum threshold can be between 10-15 lpm. In a configuration, the minimum threshold can be between 15-20 lpm. In a configuration, the minimum threshold can be between 20-25 lpm. In a configuration, the minimum threshold can be between 25-30 lpm. In a configuration, the minimum threshold can be between 30-35 lpm. In a configuration, the minimum threshold can be between 35-40 lpm. In a configuration, the minimum threshold can be between 40-45 lpm. In a configuration, the minimum threshold can be between 45-50 lpm. In a configuration, the minimum threshold can be between 50-55 lpm. In a configuration, the minimum threshold can be between 55-60 lpm.

In a configuration, the maximum threshold can be between 20-25 lpm. In a configuration, the maximum threshold can be between 25-30 lpm. In a configuration, the maximum threshold can be between 30-35 lpm. In a configuration, the maximum threshold can be between 35-40 lpm. In a configuration, the maximum threshold can be between 40-45 lpm. In a configuration, the maximum threshold can be between 45-50 lpm. In a configuration, the maximum threshold can be between 50-55 lpm. In a configuration, the maximum threshold can be between 55-60 lpm. In a configuration, the maximum threshold can be between 60-65 lpm. In a configuration, the maximum threshold can be between 65-70 lpm. In a configuration, the maximum threshold can be between 70-75 lpm. In a configuration, the maximum threshold can be between 75-80 lpm. In a configuration, the maximum threshold can be between 80-85 lpm. In a configuration, the maximum threshold can be between 85-90 lpm. In a configuration, the maximum threshold can be between 90-95 lpm. In a configuration, the maximum threshold can be between 95-100 lpm.

In a configuration, the maximum and/or minimum threshold can be a safety limit of the respiratory therapy.

In a configuration, the maximum and/or minimum threshold can depend on a selected flow rate.

In a configuration, the maximum and/or minimum threshold can be a predetermined percentage or value above and below the selected flow rate.

In a configuration, the maximum and/or minimum threshold can depend on a selected flow rate and a selected breath synchronization setting.

In a configuration, the system can comprise a user interface configured to receive a user input to adjust the breath synchronization setting.

In a configuration, the breath synchronization setting can comprise a range of numbers. In a configuration, the breath synchronization setting can comprise a plurality of categories including at least low and high settings.

In a configuration, the maximum and/or minimum threshold can be a predetermined percentage or value above and below the selected flow rate, the percentage or value varying based on the selected breath synchronization setting.

In a configuration, the adjusting can be performed iteratively.

In a configuration, the adjusting can comprise an increase in the flow rate when the patient is inspiring.

In a configuration, the adjusting can comprise a decrease in the flow rate when the patient is expiring.

In a configuration, the one or more sensors can comprise an ultrasonic transducer assembly.

In a configuration, the one or more sensors can comprise a heated temperature sensing element.

In a configuration, the system can be a high flow respiratory system.

In a configuration, the system can comprise a non-sealed patient interface.

In a configuration, the non-sealed patient interface can comprise a non-sealed nasal cannula.

In a configuration, a method of adjusting a flow rate of gases delivered to a patient according to patient inspiration and expiration using a respiratory system comprising a flow generator configured to generate the flow rate of gases can comprise using a controller of the respiratory system: determining a cycle of the patient inspiration and expiration based on information received from the one or more sensors; and adjusting the flow rate based at least in part on the cycle of the patient inspiration and expiration, wherein the adjusting can be attenuated by comparing a maximum and/or minimum flow rate measured by one or more sensors with a maximum and/or minimum threshold.

In a configuration, the flow generator can comprise a motor.

In a configuration, the flow rate can be adjusted by outputting a motor control signal.

In a configuration, the motor can comprise a brushless DC motor.

In a configuration, the determining can be based on a first input and a second inputs received by the controller, the first and second inputs relating to a gases flow characteristic or performance of a component of the system.

In a configuration, the first input can correspond to the flow rate from the one or more sensors.

In a configuration, the second input can correspond to a pressure of a gases flow from the one or more sensors. In a configuration, the second input can correspond to a speed of the motor.

In a configuration, the method can further comprise determining the speed of the motor based at least in part upon one or more motor parameters.

In a configuration, the maximum and/or minimum threshold can be set by a user.

In a configuration, the system can comprises a user interface configured to receive a user input to adjust the maximum and/or minimum threshold.

In a configuration, the maximum and/or minimum threshold can be a clinically relevant limit of the respiratory therapy.

In a configuration, the minimum threshold can be between 0-5 lpm. In a configuration, the minimum threshold can be between 10 lpm. In a configuration, the minimum threshold can be between 10-15 lpm. In a configuration, the minimum threshold can be between 15-20 lpm. In a configuration, the minimum threshold can be between 20-25 lpm. In a configuration, the minimum threshold can be between 25-30 lpm. In a configuration, the minimum threshold can be between 30-35 lpm. In a configuration, the minimum threshold can be between 35-40 lpm. In a configuration, the minimum threshold can be between 40-45 lpm. In a configuration, the minimum threshold can be between 45-50 lpm. In a configuration, the minimum threshold can be between 50-55 lpm. In a configuration, the minimum threshold can be between 55-60 lpm.

In a configuration, the maximum threshold can be between 20-25 lpm. In a configuration, the maximum threshold can be between 25-30 lpm. In a configuration, the maximum threshold can be between 30-35 lpm. In a configuration, the maximum threshold can be between 35-40 lpm. In a configuration, the maximum threshold can be between 40-45 lpm. In a configuration, the maximum threshold can be between 45-50 lpm. In a configuration, the maximum threshold can be between 50-55 lpm. In a configuration, the maximum threshold can be between 55-60 lpm. In a configuration, the maximum threshold can be between 60-65 lpm. In a configuration, the maximum threshold can be between 65-70 lpm. In a configuration, the maximum threshold can be between 70-75 lpm. In a configuration, the maximum threshold can be between 75-80 lpm. In a configuration, the maximum threshold can be between 80-85 lpm. In a configuration, the maximum threshold can be between 85-90 lpm. In a configuration, the maximum threshold can be between 90-95 lpm. In a configuration, the maximum threshold can be between 95-100 lpm.

In a configuration, the maximum and/or minimum threshold can be a safety limit of the respiratory therapy.

In a configuration, the maximum and/or minimum threshold can depend on a selected flow rate.

In a configuration, the maximum and/or minimum threshold can be a predetermined percentage or value above and below the selected flow rate.

In a configuration, the maximum and/or minimum threshold can depend on a selected flow rate and a selected breath synchronization setting.

In a configuration, the system can comprise a user interface configured to receive a user input to adjust the breath synchronization setting.

In a configuration, the breath synchronization setting can comprise a range of numbers. In a configuration, the breath synchronization setting can comprise a plurality of categories including at least low and high settings.

In a configuration, the maximum and/or minimum threshold can be a predetermined percentage or value above and below the selected flow rate, the percentage or value varying based on the selected breath synchronization setting.

In a configuration, the adjusting can be performed iteratively.

In a configuration, each adjusting can be bound by a parameter determined based in part on a maximum and/or minimum flow rate measured by the one or more sensors.

In a configuration, the maximum and/or minimum flow rate measured by the one or more sensors can be an unbounded response to the patient inspiration and expiration.

In a configuration, the maximum and/or minimum flow rate measured by the one or more sensors can be gradually bounded by a negative feedback term.

In a configuration, the parameter can comprise the negative feedback term that can be gradually increased in response to the measured maximum and/or minimum flow rates until the measured maximum and/or minimum flow rate no longer exceeds the maximum and/or minimum threshold.

In a configuration, the adjusting can comprise an increase in the flow rate when the patient is inspiring.

In a configuration, the adjusting can comprise a decrease in the flow rate when the patient is expiring.

In a configuration, the one or more sensors can comprise an ultrasonic transducer assembly.

In a configuration, the one or more sensors can comprise a heated temperature sensing element.

In a configuration, the system can be a high flow respiratory system.

In a configuration, the system can comprise a non-sealed patient interface.

In a configuration, the non-sealed patient interface can comprise a non-sealed nasal cannula.

In a configuration, a respiratory system for delivering a respiratory therapy to a patient, the system configured to adjust a flow rate of gases delivered to the patient according to patient inspiration and expiration, can comprise a flow generator with a motor for generating a gases flow; and a controller in electrical communication with one or more sensors and configured to: cause to be displayed, on a user interface of the respiratory system, an expiratory relief level setting; receive a first user input to increase and/or decrease an expiratory relief level; cause to be displayed, on the user interface, a maximum and/or minimum flow rate threshold setting; receive a second user input to increase and/or decrease the maximum and/or minimum flow rate threshold; and adjust the flow rate based on the first user input and the second user input, wherein the first user input can be configured to allow adjusting of the flow rate based in part on a cycle of the patient inspiration and expiration and the second user input can be configured to attenuate the adjusting based on the first user input by comparing a maximum and/or minimum flow rate measured by the one or more sensors with the maximum and/or minimum threshold.

In a configuration, the first user input can be configured to allow adjusting of the flow rate based in part on a cycle of the patient inspiration and expiration by turning on and/or off the expiratory relief, and/or adjusting a magnitude of the expiratory relief.

In a configuration, the expiratory relief level setting can comprise a plurality of different expiratory relief levels.

In a configuration, the plurality of different expiratory relief levels can comprise a plurality of categories including at least low and high expiratory relief levels. In a configuration, the plurality of different expiratory relief levels can comprise a range of numbers. In a configuration, the plurality of different expiratory relief levels can comprise a sliding scale.

In a configuration, the first user input can be received via button(s) on the user interface.

In a configuration, the expiratory relief level setting can be accessible by the patient.

In a configuration, the maximum and/or minimum flow rate threshold setting may not be accessible by the patient.

In a configuration, the maximum and/or minimum flow rate threshold setting can be accessible by a clinician or technician.

In a configuration, the system can be a high flow respiratory system.

In a configuration, the system can comprise a non-sealed patient interface.

In a configuration, the non-sealed patient interface can comprise a non-sealed nasal cannula.

In a configuration, the expiratory relief levels can affect a negative feedback to adjusting of the flow rate as applied in any of the configurations disclosed herein.

In a configuration, a respiratory system for delivering a respiratory therapy to a patient, the system configured to adjust a flow rate of gases delivered to the patient according to patient inspiration and expiration, can comprise a flow generator with a motor for generating a gases flow; and a controller in electrical communication with one or more sensors and configured to: cause to be displayed, on a user interface of the respiratory system, an expiratory relief level setting, the setting comprising a plurality of different expiratory relief levels; receive a user input via the user interface to increase and/or decrease an expiratory relief level; and adjust the flow rate based on the user input, wherein the user input can be configured to allow adjusting of the flow rate based in part on a cycle of the patient inspiration and expiration.

In a configuration, the user input can be configured to allow adjusting of the flow rate based in part on a cycle of the patient inspiration and expiration by turning on and/or off the expiratory relief, and/or adjusting a magnitude of the expiratory relief.

In a configuration, the plurality of different expiratory relief levels can comprise a plurality of categories including at least low and high expiratory relief levels. In a configuration, the plurality of different expiratory relief levels can comprise a range of numbers. In a configuration, the plurality of different expiratory relief levels can comprise a sliding scale.

In a configuration, the first user input can be received via button(s) on the user interface.

In a configuration, the controller can be further configured to attenuate the adjusting based on the user input by comparing a maximum and/or minimum flow rate measured by the one or more sensors with a maximum and/or minimum flow rate threshold.

In a configuration, the system can be a high flow respiratory system.

In a configuration, the system can comprise a non-sealed patient interface.

In a configuration, the non-sealed patient interface can comprise a non-sealed nasal cannula.

In a configuration, the expiratory relief levels can affect a negative feedback to adjusting of the flow rate as applied in any of the configurations disclosed herein.

In a configuration, a respiratory system for delivering a respiratory therapy to a patient, the system configured to adjust a flow rate of gases delivered to the patient according to patient inspiration and expiration, can comprise a flow generator with a motor for generating a gases flow; and a controller in electrical communication with one or more sensors and configured to: cause to be displayed, on the user interface, a maximum and/or minimum flow rate threshold setting; receive a user input to increase and/or decrease the maximum and/or minimum flow rate threshold; and adjust the flow rate based on the user input, wherein the user input can be configured to attenuate adjusting of the flow rate based on a cycle of the patient inspiration and expiration by comparing a maximum and/or minimum flow rate measured by the one or more sensors with the maximum and/or minimum threshold.

In a configuration, the user input can be received via button(s) on the user interface.

In a configuration, the maximum and/or minimum flow rate threshold setting may not be accessible by the patient.

In a configuration, the maximum and/or minimum flow rate threshold setting can be accessible by a clinician or technician.

In a configuration, the maximum and/or minimum flow rate threshold can affect a negative feedback term to adjusting of the flow rate as applied in any of the configurations disclosed herein.

In a configuration, the controller can be configured to adjust the flow rate based on the cycle of the patient inspiration and expiration by receiving an expiratory relief level set by a user.

In a configuration, the controller can be configured to cause to be displayed, on the user interface, a plurality of different expiratory relief levels.

In a configuration, the plurality of different expiratory relief levels can comprise a plurality of categories including at least low and high expiratory relief levels. In a configuration, the plurality of different expiratory relief levels can comprise a range of numbers. In a configuration, the plurality of different expiratory relief levels can comprise a sliding scale.

In a configuration, the expiratory relief level setting can be accessible by the patient.

In a configuration, the system can be a high flow respiratory system.

In a configuration, the system can comprise a non-sealed patient interface.

In a configuration, the non-sealed patient interface can comprise a non-sealed nasal cannula.

BRIEF DESCRIPTION OF THE DRAWINGS

These and other features, aspects, and advantages of the present disclosure are described with reference to the drawings of certain embodiments, which are intended to schematically illustrate certain embodiments and not to limit the disclosure.

Figure 1A illustrates schematically a high flow respiratory system configured to provide a respiratory therapy to a patient.

Figure 1B is a front perspective view of an example high flow therapy apparatus with a humidification chamber in position.

Figure 1C is a back perspective view of the high flow therapy apparatus of Figure 1B.

Figure 2A illustrates an example block diagram of a control system interacting with and/or providing control and direction to components of a flow therapy apparatus.

Figure 2B illustrates an example block diagram of a control system interacting with and/or providing control and direction to components of a flow therapy apparatus.

Figure 2C illustrates a block diagram of an example controller.

Figure 3A illustrates a block diagram of an example motor/sensor module.

Figure 3B illustrates an example sensing chamber of a flow therapy apparatus.

Figure 4 illustrates a flowchart of an example process for adjusting the operation of a flow therapy apparatus.

Figure 5 illustrates a block diagram of an example system for performing breath cycle enhancement for a flow therapy apparatus.

Figure 6 illustrates example motor speed and flow rate of a flow therapy apparatus during breath cycle enhancement.

Figures 7A-7C illustrate example user interface displays or portions thereof showing target flow rate and/or expiratory relief settings.

Figure 8 shows in diagrammatic form another flow therapy system.

Figure 9 is a schematic diagram of a closed loop control system.

DETAILED DESCRIPTION

Although certain examples are described below, those of skill in the art will appreciate that the disclosure extends beyond the specifically disclosed examples and/or uses and obvious modifications and equivalents thereof. Thus, it is intended that the scope of the disclosure herein disclosed should not be limited by any particular examples described below.

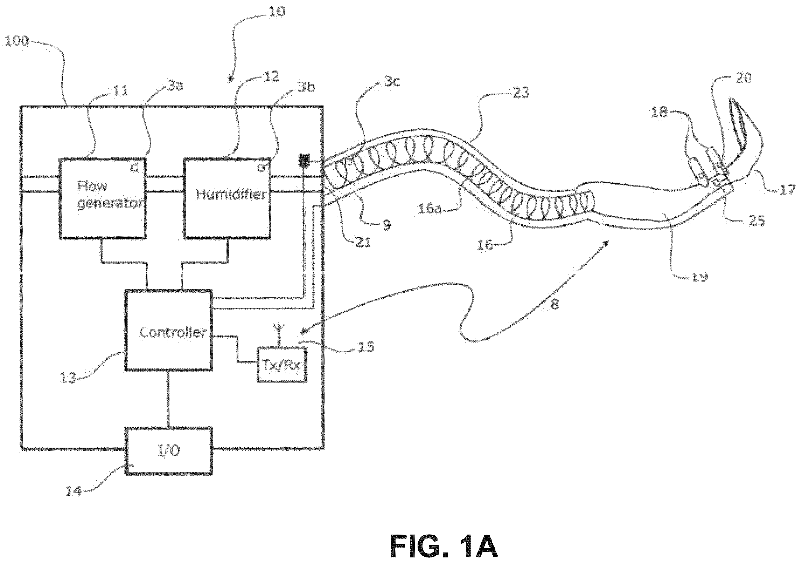

A schematic representation of a high flow respiratory system 10 is provided in Figure 1A. The respiratory system 10 can include a main device housing 100. The main device housing 100 can contain a flow generator 11 that can be in the form of a motor/impeller arrangement (such as a blower), an optional humidifier or humidification chamber 12, a controller 13, and a user interface 14. The user interface 14 can include a display and input device(s) such as button(s), a touch screen, a combination of a touch screen and button(s), or the like. The controller 13 can include one or more hardware and/or software processors and can be configured or programmed to control the components of the apparatus, including but not limited to operating the flow generator 11 to create a flow of gas for delivery to a patient, operating the humidifier 12 (if present) to humidify and/or heat the gas flow, receiving user input from the user interface 14 for reconfiguration and/or user-defined operation of the respiratory system 10, and outputting information (for example on the display) to the user. The user can be a patient, healthcare professional, or anyone using the system 10.

With continued reference to Figure 1A, a patient breathing conduit 16 can be coupled to a gases flow outlet 21 in the main device housing 100 of the respiratory system 10, and be coupled to a patient interface 17. The patient interface can be a non-sealing interface like a nasal cannula with a manifold 19 and nasal prongs 18 for providing a high flow therapy. The patient breathing conduit 16 can also be coupled to a sealing interface like a face mask, a nasal mask, or a nasal pillow mask. The patient interface can also optionally include an endotracheal tube, a tracheostomy interface, or others.

The flow of gas can be generated by the flow generator 11, and may be humidified, before being delivered to the patient via the patient conduit 16 through the patient interface 17. The controller 13 can control the flow generator 11 to generate a gas flow of a desired flow rate, and/or one or more valves to control mixing of air and oxygen or other breathable gas. The controller 13 can control a heating element in the humidification chamber 12, if present, to heat the gases to a desired temperature that achieves a desired level of temperature and/or humidity for delivery to the patient. The patient conduit 16 can have a heating element 16a, such as a heater wire, to heat gases flow passing through to the patient. The heating element 16a can also be under the control of the controller 13.

The system 10 can use flow rate sensor(s), pressure sensor(s), temperature sensor(s), humidity sensor(s), or other sensors, in communication with the controller 13, to monitor characteristics of the gas flow and/or operate the system 10 in a manner that provides suitable therapy. Ultrasonic transducers and heated temperature sensing elements are examples of sensors that can be used to measure flow rate, among other parameters. The gas flow characteristics can include gases concentration, flow rate, pressure, temperature, humidity, or others. The sensors 3a, 3b, 3c, 20, 25, such as pressure, temperature, humidity, and/or flow rate sensors, can be placed in various locations in the main device housing 100, the patient conduit 16, and/or the patient interface 17. The controller 13 can receive output from the sensors to assist it in operating the respiratory system 10 in a manner that provides suitable therapy, such as to determine a suitable target temperature, flow rate, and/or pressure of the gases flow. Providing suitable therapy can include meeting a patient's inspiratory demand.

The system 10 can include a wireless data transmitter and/or receiver, or a transceiver 15 to enable the controller 13 to receive data signals 8 in a wireless manner from the operation sensors and/or to control the various components of the system 10. Additionally, or alternatively, the data transmitter and/or receiver 15 can deliver data to a remote server or enable remote control of the system 10. The system 10 can also include a wired connection, for example, using cables or wires, to enable the controller 13 to receive data signals 8 from the operation sensors and/or to control the various components of the system 10.

As used herein, "high flow" therapy refers to administration of gas to the airways of a patient at a relatively high flow rate that generally meets or exceeds the peak inspiratory demand of the patient. The flow rates used to achieve "high flow" may be any of the flow rates listed below. For example, in some configurations, for an adult patient 'high flow therapy' may refer to the delivery of gas(es) to a patient at a flow rate of greater than or equal to about 10 litres per minute (10 LPM), such as between about 10 LPM and about 100 LPM, or between about 15 LPM and about 95 LPM, or between about 20 LPM and about 90 LPM, or between about 25 LPM and about 85 LPM, or between about 30 LPM and about 80 LPM, or between about 35 LPM and about 75 LPM, or between about 40 LPM and about 70 LPM, or between about 45 LPM and about 65 LPM, or between about 50 LPM and about 60 LPM. In some configurations, for a neonatal, infant, or child patient 'high flow therapy' may refer to the delivery of gas(es) to a patient at a flow rate of greater than 1 LPM, such as between about 1 LPM and about 25 LPM, or between about 2 LPM and about 25 LPM, or between about 2 LPM and about 5 LPM, or between about 5 LPM and about 25 LPM, or between about 5 LPM and about 10 LPM, or between about 10 LPM and about 25 LPM, or between about 10 LPM and about 20 LPM, or between about 10 LPM and 15 LPM, or between about 20 LPM and 25 LPM. A high flow therapy apparatus with an adult patient, a neonatal, infant, or child patient, may deliver gas(es) to the patient at a flow rate of between about 1 LPM and about 100 LPM, or at a flow rate in any of the sub-ranges outlined above.

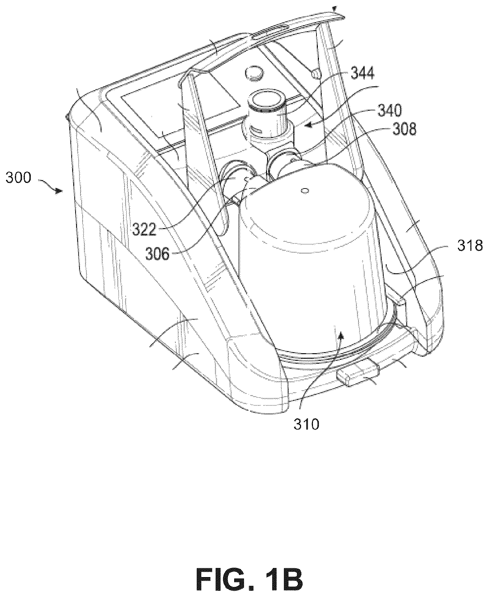

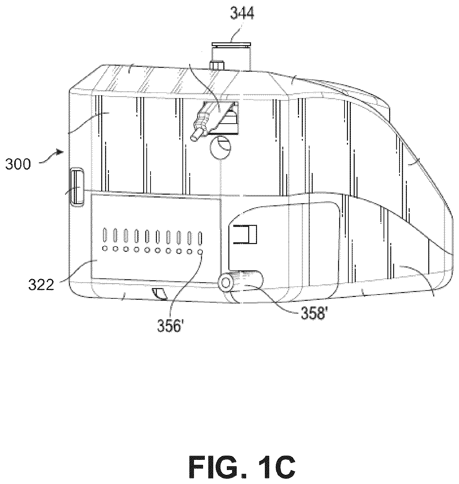

Figures 1B and 1C show an example flow therapy apparatus or respiratory device of the respiratory system 10. The device can include a housing 300, which encloses a flow generator. The flow generator may include a motor/sensor module. The motor/sensor module may be non-removable from the main housing 300. The motor/sensor module can also optionally be removable from the main housing 300. The housing 300 can include a humidifier or humidification chamber bay 318 for receipt of a removable humidification chamber 310. The removable humidification chamber 310 contains a suitable liquid such as water for heating and humidifying gases delivered to a patient. The humidification chamber 310 can be fluidly coupled to the device housing 300 in a linear slide-on motion into the chamber bay 318. A gas outlet port 322 can establish a fluid communication between the motor/sensor module and an inlet 306 of the chamber 310.

Heated and humidified gas can exit an outlet 308 of the chamber 310 into a humidified gas return 340, which can include a removable L-shaped elbow. The removable elbow can further include a patient outlet port 344 for coupling to the inspiratory conduit, such as the inspiratory conduit 16 of Figure 1A to deliver gases to the patient interface 17. The gas outlet port 322, humidified gas return 340, and patient outlet port 344 each can have seals such as O-ring seals or T-seals to provide a sealed gases passageway between the device housing 300, the humidification chamber 310, and the inspiratory conduit. A floor portion of the humidification chamber bay 318 in the housing 300 can include a heater arrangement such as a heater plate or other suitable heating element(s) for heating the water in the humidification chamber 310 for use during a humidification process.

As shown in Figure 1C, the device can include an arrangement to enable the flow generator to deliver air, oxygen (or alternative auxiliary gas), or a suitable mixture thereof to the humidification chamber 310 and thereby to the patient. This arrangement can include an air inlet 356' in a rear wall 322 of the housing 300. The device can include a separate oxygen (or other breathable gases) inlet port 358'. In the illustrated configuration, the oxygen inlet port 358' can be positioned adjacent one side of the housing 300 at a rear end thereof. The oxygen port 358' can be connected to an oxygen source such as a tank. The oxygen inlet port 358' can be in fluid communication with a valve. The valve can suitably be a solenoid valve that enables the control of the amount of oxygen that is added to the gas flow that is delivered to the humidification chamber 310.

The housing 300 can include suitable electronics boards, such as sensing circuit boards. The electronics boards can contain, or can be in electrical communication with, suitable electrical or electronics components, such as but not limited to microprocessors, capacitors, resistors, diodes, operational amplifiers, comparators, and switches. One or more sensors can be used with the electronic boards. Components of the electronics boards (such as but not limited to one or more microprocessors) can act as the controller 13 of the apparatus. One or both of the electronics boards can be in electrical communication with the electrical components of the system 10, including but not limited to the display unit and user interface 14, motor, valve, and the heater plate to operate the motor to provide the desired flow rate of gases, humidify and heat the gases flow to an appropriate level, and supply appropriate quantities of oxygen (or quantities of an alternative auxiliary gas) to the gases flow.

As mentioned above, operation sensors, such as flow, temperature, humidity, and/or pressure sensors can be placed in various locations in the respiratory device, the patient conduit 16, and/or cannula 17. The electronics boards can be in electrical communication with those sensors. Output from the sensors can be received by the controller 13, to assist the controller 13 to operate the respiratory system 10 in a manner that provides optimal therapy, including generally meeting inspiratory demand. One or more sensors (for example, Hall-effect sensors) may be used to measure a motor speed of the motor of the flow generator. The motor may include a brushless DC motor, from which motor speed can be measured without the use of separate sensors. For example, during operation of a brushless DC motor, back-EMF can be measured from the non-energized windings of the motor, from which a motor position can be determined, which can in turn be used to calculate a motor speed. In addition, a motor driver may be used to measure motor current, which can be used with the measured motor speed to calculate a motor torque. The motor may also include a low inertia motor.

Room air can enter the flow generator through the inlet port, such as the air inlet port 356' in Figure 1C. The flow generator can operate at a motor speed of greater than 1,000 RPM and less than 30,000 RPM, greater than 2,000 RPM and less than 21,000 RPM, or between any of the foregoing values. Operation of the flow generator can mix the gases entering the flow generator, such as the motor/sensor chamber through the inlet port. Using the flow generator as the mixer can reduce the pressure drop that would otherwise occur in a system with a separate mixer, such as a static mixer comprising baffles, because mixing requires energy.

Control System

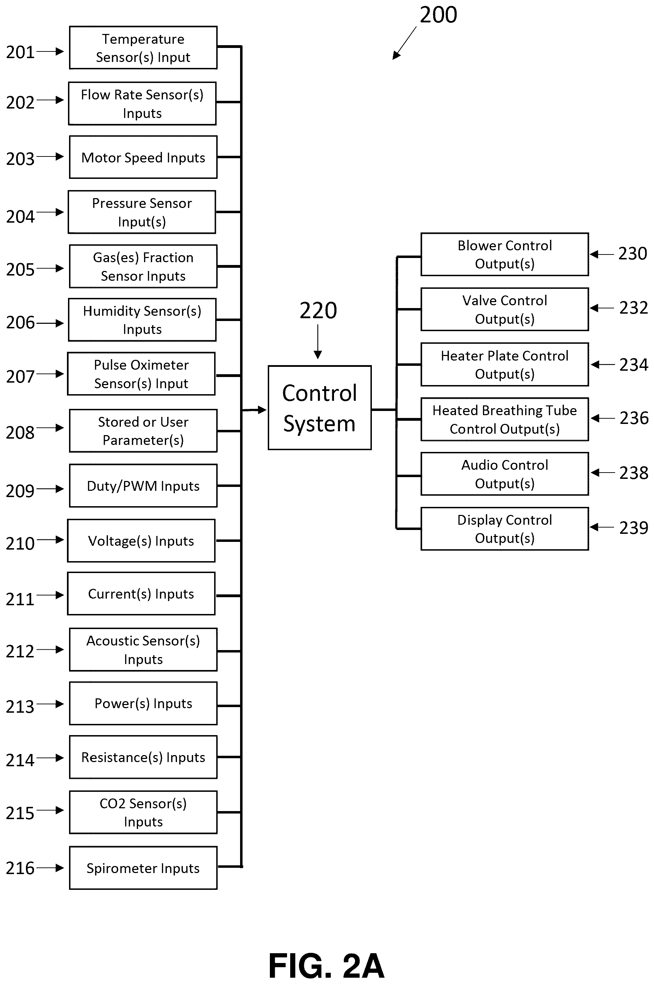

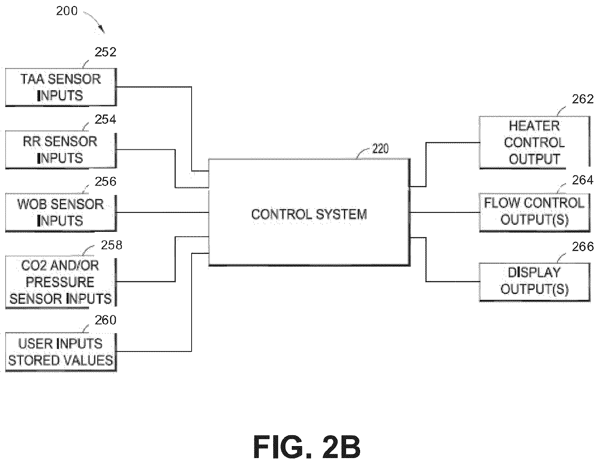

Figures 2A and 2B illustrate a block diagram 200 of an example control system 220 that can detect patient conditions and control operation of the flow therapy apparatus including the gas source. The control system 220 can manage a flow rate of the gas flowing through the flow therapy apparatus as it is delivered to a patient. For example, the control system 220 can increase or decrease the flow rate by controlling an output of motor speed of the flow generator or blower 230 or an output of a valve 232 (such as in an auxiliary gas port). The control system 220 can automatically determine a set value or a personalized value of the flow rate for a particular patient as discussed below. The flow rate can be optimized by the control system 220 to improve patient comfort and therapy.

The control system 220 can generate audio and/or display/visual outputs 238, 239. For example, the flow therapy apparatus can include a display and/or a speaker. The display can indicate to the physicians any warnings or alarms generated by the control system 220. The display can also indicate control parameters that can be adjusted by the physicians. For example, the control system 220 can automatically recommend a flow rate for a particular patient. The control system 220 can also determine a respiratory state of the patient, including but not limited to generating a respiratory rate of the patient, and send it to the display.

The control system 220 can change heater control outputs to control one or more of the heating elements (for example, to maintain a temperature set point of the gas delivered to the patient). The control system 220 can also change the operation or duty cycle of the heating elements. The heater control outputs can include heater plate control output(s) 234 and heated breathing tube control output(s) 236.

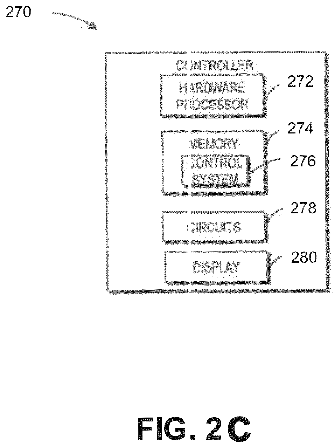

The control system 220 can determine the outputs 230-239 based on one or more received inputs 201-216. The inputs 201-216 can correspond to sensor measurements received automatically by the controller (shown in Figures 1A, 2B and 2C). The control system 220 can receive sensor inputs including but not limited to temperature sensor(s) inputs 201, flow rate sensor(s) inputs 202, motor speed inputs 203, pressure sensor(s) inputs 204, gas(s) fraction sensor(s) inputs 205, humidity sensor(s) inputs 206, pulse oximeter (for example, $SpO_2$) sensor(s) inputs 207, stored or user parameter(s) 208, duty cycle or pulse width modulation (PWM) inputs 209, voltage(s) inputs 210, current(s) inputs 211, acoustic sensor(s) inputs 212, power(s) inputs 213, resistance(s) inputs 214, $CO_2$ sensor(s) inputs 215, and/or spirometer inputs 216. The control system 220 can receive inputs from the user or stored parameter values in a memory 304 (shown in Figure 2C). The control system 220 can dynamically adjust flow rate for a patient over the time of their therapy. The control system 220 can continuously detect system parameters and patient parameters. Any other suitable inputs and/or outputs can be used with the control system 220. For example, with the pulse oximeter sensor(s) input 207, the control system 220 can implement one or more closed loop control systems to control the composition of oxygen in the flow of gases, such as described in International application No. PCT/NZ2018/050137, filed October 5, 2018 and published as WO2019/070136. With the closed loop control system(s), the flow therapy apparatus can monitor blood oxygen saturation ($SpO_2$) of a patient and control the fraction of oxygen delivered to the patient ($FdO_2$). The flow therapy apparatus can also automatically adjust the $FdO_2$ in order to achieve a targeted $SpO_2$ value for the patient.

As illustrated in Figure 2B, the control system 220 can receive inputs from multiple components of the flow therapy apparatus, such as thoraco-abdominal asynchrony (TAA) sensor inputs 252, respiratory sensor inputs 254, work of breathing (WOB) sensor inputs 256, $CO2$ and/or pressure sensor inputs 258, user inputs and/or stored values 260. Not all of the inputs 202-210 shown in Figure 2A may be present. The control system 220 in Figure 2B can output based on the inputs 252-260 heater control output 262, flow control output(s) 264, and display/audio output(s) 266. The inputs 202 to 210 and the outputs 230 to 234 may not all necessarily be present. For example, the control system 220 may only receive the WOB sensor (such as EMG) input 256 and generate a flow control measurement 264. Depending on the configuration, some of the components corresponding to the inputs may not be included in the flow therapy apparatus. Lack of input itself can be used by the control system 220 to determine the input or system conditions.

The control system 220 can include programming instructions for detection of input conditions and control of output conditions. Figure 2C illustrates a block diagram of an example controller 270. The programming instructions can be stored in a memory 274 of the controller 270. The programming instructions can correspond to the methods, processes and functions described herein. The control system 276 can be executed by one or more hardware processors 272 of the controller 270. The programming instructions can be implemented in C, C++, JAVA, or any other suitable programming languages. The controller can also include circuits 278 for receiving sensor signals. Some or all of the portions of the control system 276 can be implemented in application specific circuitry 278 such as ASICs and FPGAs.

The controller can further include a display 280 for transmitting status of the patient and the respiratory assistance system. The display 280 can also show warnings. The controller can also receive user inputs via the user interface such as the display 280. The user interface may alternatively or additionally include buttons or a dial.

Motor/Sensor Module

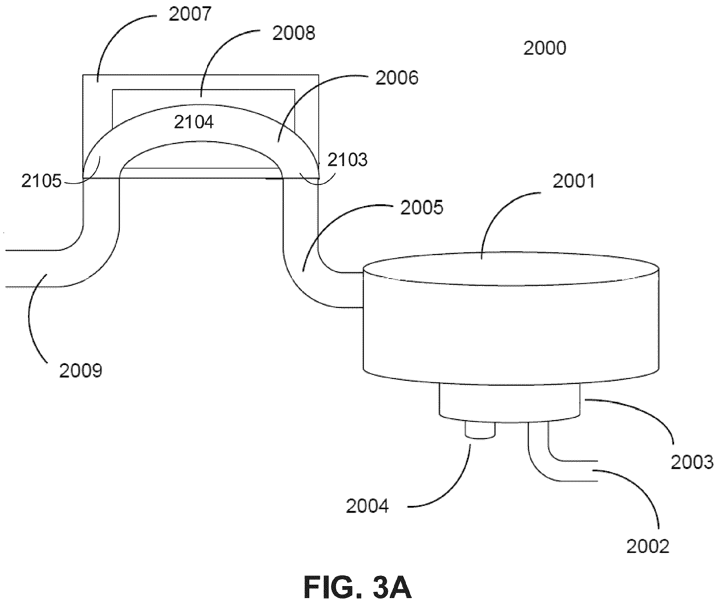

Figure 3A illustrates a block diagram of a motor/sensor module 2000 which may be used as part of the flow therapy apparatus. The motor/sensor module includes a flow generator 2001, which entrains room air to deliver to a patient. The flow generator 2001 can be a centrifugal blower.

Room air enters a room air inlet 2002, which enters the flow generator 2001 through an inlet port 2003. The inlet port 2003 can include a valve 2004 through which a pressurized gas may enter the flow generator 2001. The valve 2004 can control a flow of oxygen (or other auxiliary gases) into the blower 2001. The valve 2004 can be any type of valve, including a proportional valve or a binary valve. The inlet port can include no valves.

The flow generator 2001 can operate at a motor speed of greater than 1,000 RPM and less than 30,000 RPM, greater than 2,000 RPM and less than 25,000 RPM, greater than 3,000 RPM and less than 24,000 RPM, or between any of the foregoing values. Operation of the flow generator 2001 mixes the gases entering the flow generator 2001 through the inlet port 2003. Using the flow generator 2001 as the mixer can decrease the pressure drop that would otherwise occur in a system with a separate mixer, such as a static mixer comprising baffles, because mixing requires energy whereas the flow generator imparts energy.

The mixed air exits the flow generator 2001 through a conduit 2005 and enters the flow path 2006 in the sensing chamber 2007. A circuit board with sensors 2008 is positioned in the sensing chamber 2007 such that the circuit board is immersed in the gas flow. The sensors 2008 on the circuit board are positioned within the gas flow to measure gas properties within the flow. After passing through the flow path 2006 in the sensing chamber 2007, the gases exit 2009 to the humidification chamber 310.

The flow path 2006 has a curved shape. The gas flow enters at an entrance 2103, flows along a curved flow path 2104, and exits on the opposite side of the flow path 2105. The entrance and exit may be positioned in vertically opposed directions, and the gas flow may enter the path in a vertical upwards direction, then curve around to a horizontal direction, and then curve around to a vertical upwards direction again. The flow path may have no sharp turns. The flow path may have curved ends with a straighter middle section. The flow path can maintain a constant cross-section shape throughout the length of the flow path. The flow path can taper inward slightly from the first end of the flow path, and widens again to the second end of the flow path, which can speed up the flow for better accuracy, stability and reproducibility in measurements. The surface of the flow path can be lined with a surface modifier/lubricant to reduce friction within the flow path. A curved flow path shape can reduce a gas flow's pressure drop without reducing the sensitivity of flow measurements by partially coinciding the measuring region with the flow path. A number of different flow path configurations can be used.

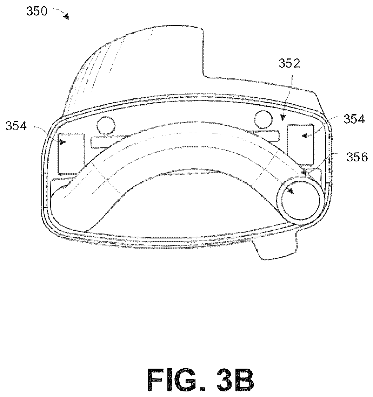

As shown in Figure 3B, the mixed air can exit the flow generator and enter a flow path 356 in a sensor chamber 350, which can be located in the motor/sensor module. A sensing circuit board 352 with sensors, such as ultrasonic transducers 354 and/or heated temperature sensing elements, can be positioned in the sensor chamber 350 such that the sensing circuit board is at least partially immersed in the gas flow. At least some of the sensors on the sensing circuit board can be positioned within the gas flow to measure gas properties within the flow. After passing through the flow path 356 in the sensor chamber 350, the gas can exit to the humidification chamber.

The sensing circuit board 352 can include sensors such as acoustic transmitters and/or receivers, humidity sensor, temperature sensor, thermistor, and the like. A gas flow rate may be measured using at least two different types of sensors. The first type of sensor can include a heated temperature sensing element (or a thermistor), which can determine a flow rate by monitoring heat transfer between the gases flow and the heated temperature sensing elements. The heated temperature sensing elements can be run at a constant target temperature within the flow when the gas flows around and past the heated temperature sensing elements. The sensor can measure an amount of power required to maintain the heated temperature sensing elements at the target temperature. The target temperature can be configured to be higher than a temperature of the gas flow, such that more power is required to maintain the heated temperature sensing elements at the target temperature at a higher flow rate.

The second type of sensor can include an acoustic (such as ultrasonic transducer) sensor assembly. Acoustic sensors including acoustic transmitters and/or receivers can be used to measure a time of flight of acoustic signals to determine gas velocity and/or composition, which can be used in flow therapy apparatuses. In one ultrasonic sensing (including ultrasonic transducers, which can act as transmitters and/or receivers) topology, a driver causes a first sensor, such as an ultrasonic transducer, to produce an ultrasonic pulse in a first direction. A second sensor, such as a second ultrasonic transducer, receives this pulse and provides a measurement of the time of flight of the pulse between the first and second ultrasonic transducers. Using this time of flight measurement, the speed of sound of the gas flow between the ultrasonic transducers can be calculated by a processor or controller of the flow therapy apparatus. The second sensor can also transmit and the first sensor can receive a pulse in a second direction opposite the first direction to provide a second measurement of the time of flight, allowing characteristics of the gas flow, such as a flow rate or velocity, to be determined. In another acoustic sensing topology, acoustic pulses transmitted by an acoustic transmitter, such as an ultrasonic transducer, can be received by acoustic receivers, such as microphones.

Readings from both the first and second types of sensors can be combined to determine a more accurate flow measurement. For example, a previously determined flow rate and one or more outputs from one of the types of sensor can be used to determine a predicted current flow rate. The predicted current flow rate can then be updated using one or more outputs from the other one of the first and second types of sensor, in order to calculate a final flow rate.

Adjusting Flow Based Upon Breath Cycle

Some patients may find it more comfortable to adjust the operation of a flow therapy apparatus based upon the patient's breath cycle. For example, as a patient inhales and exhales, a flow rate of air provided by the flow therapy apparatus may be adjusted. The flow rate may be increased during the patient's inspiration, and decreased during the patient's expiration. The flow rate may be adjusted during a patient's inspiration (for example, increased during inspiration), with no adjustment during the patient's expiration, or vice versa. Inspiration and expiration may also be referred to as inhalation and exhalation.

A patient's breathing cycle may be represented as a waveform comprising alternating inhalation and exhalation phases. By determining and monitoring a patient's breath cycle waveform, operations of the flow therapy apparatus can be modified based upon the patient's breath cycle. For example, the flow therapy apparatus may be configured to control a gas flow using a periodic waveform, which may be adjusted based upon the patient's measured breath cycle waveform.

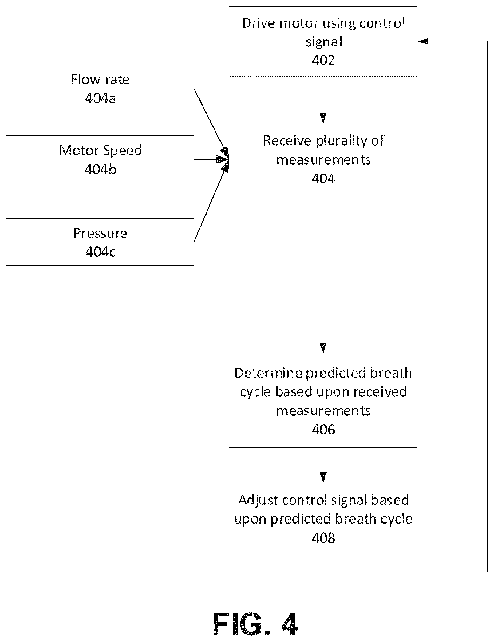

Figure 4 illustrates a flowchart of an example process for adjusting the operation of a flow therapy apparatus. At block 402, a control signal is used to drive a motor associated with the flow therapy apparatus (for example, flow generator 11 as illustrated in Figure 1A). The motor may be used to generate an air flow in order to assist the respiration of a patient. The control signal may include an initial waveform. The initial waveform may include a default waveform, or be based upon one or more measurements associated with the patient.

At block 404, a plurality of measurements are received at the controller that may be used to determine a breathing cycle of the patient. These may include a flow rate 404a, a motor speed 404b, a pressure 404c, and/or the like.

At block 406, the received measurements are used to determine a predicted breath cycle of the patient. The predicted breath cycle of the patient may be determined using one or more different techniques, such as by monitoring flow deviation (for example, from an average or set-point flow rate value), flow restriction (for example, as discussed below), system leak (that is, a portion of the flow of air generated by a blower that did not flow to a patient's lungs), and/or the like.

With continued reference to Figure 4, at block 408, the control signal to the motor is adjusted based upon the predicted breath cycle. For example, the control signal may be adjusted so that the flow rate is increased as the patient inhales, and decreased as the patient exhales.

The process may then return to block 402, where the adjusted control signal is used to drive the blower motor to produce an air flow for the patient.

The process can also be implemented on a respiratory system with a sealed patient interface. The pressure sensor can be placed anywhere in the flow path. A non-limiting example of a sealed patient interface is an NIV mask. NIV masks can be sealed against the patient's face, resulting in substantially no system leak. This makes it possible to measure the pressure of the gases delivered to the patient near or at the patient end. For example, the pressure sensor can be positioned inside the NIV mask or at a location outside the patient's nares.

The pressure sensor can be positioned in a manifold connecting the NIV mask to the patient breathing conduit, such as the patient breathing conduit 16 shown in Figure 1A.

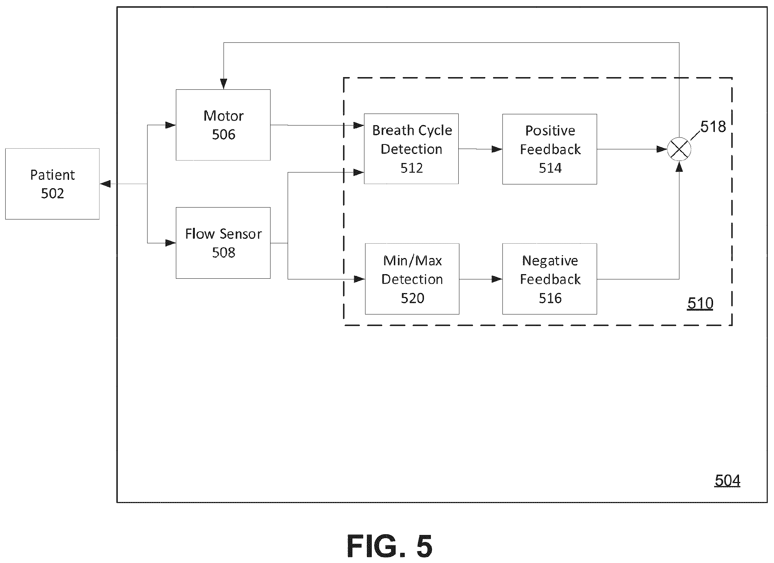

Figure 5 illustrates a block diagram of an example system for adjusting the control signal to the motor at block 408 of Figure 4. As illustrated in Figure 5, a patient 502 is connected to a flow therapy apparatus 504, such as the flow therapy apparatus 10 of Figure 1A. The apparatus can include a flow generator with a motor 506, which may be used to provide an air flow to the patient 502.

During operation of the flow therapy apparatus 504, a plurality of measurements may be taken and transmitted to a control signal feedback module 510, in order to adjust a control signal to the motor 506 based upon a breath cycle of the patient 502. For example, parameters of the motor 506 may be used to measure a motor speed as described above and/or a system pressure. A flow rate of the air flow may be monitored using one or more flow rate sensors 508. The flow rate sensors 508 may include two or more different types of sensors, such as a heated temperature sensing element and an ultrasonic transducer assembly. In addition, one or more additional sensors, such as pressure sensors may be used to measure one or more additional measurements (for example, pressure).

The plurality of measurements (for example, motor speed, flow rate, and/or the like) may be used to determine a breath cycle of the patient at a breath cycle detection module 512. The determined breath cycle may be in the form of an alternating waveform (for example, a substantially sinusoidal waveform). The measurements about the motor 506 and the measurements by the flow rate sensor 508 can both be fed into the breath cycle detection module 512.

Once the breath cycle of the patient has been determined, it may be used to adjust the control signal to the motor 506. For example, the calculated breath cycle waveform from the breath cycle detection module 512 may be subject to positive feedback 514.

Positive feedback 514 can function to work with the patient during the patient's breath cycle by reducing the motor speed as the patient exhales, and/or increasing motor speed as the patient inhales. Positive feedback may be implemented during inspiration but not during expiration, or positive feedback may be implemented during expiration but not during inspiration. For example, a patient that attempts to lower his or her work when breathing using "pursed lip breathing" on expiration may benefit from being assisted with positive feedback for increasing flow rate during inspiration, but no positive feedback for decreasing flow rate during expiration. By not implementing positive feedback during expiration, expiration pressure and expiration time may be increased, which may be beneficial for certain patients. One or more scaling parameters may be used to increase or decrease the magnitude of the control signal controlling the speed of motor 506, based upon a determined magnitude of the patient's inhale/exhale. For example, positive feedback for the blower motor control signal may be expressed as:

$$\omega = \overline{\omega} + k_p(R - \overline{R})$$

where $\omega$ corresponds to motor speed, $R$ corresponds to a patient restriction, $\overline{\omega}$ and $\overline{R}$ correspond to their average or baseline values, and $k_p$ corresponds to a positive feedback parameter.

As noted above, flow restriction may also be used to determine a patient's breath cycle. In general, a breathing system as a whole can have some resistance to flow (also referred to as "Restriction" or $R$), which can be used to indicate a relationship between change in pressure $p$ of the system and the flow of the system. The restriction $R$ may vary as the patient inhales and exhales. Larger values of $R$ represent larger restrictions (for example, when the patient exhales).

With continued reference to Figure 5, the measurements from the flow rate sensor 508 can also be fed into a minimum and/or maximum detection module 520 for the measured flow rate. The minimum and/or maximum measured flow rate can be used to determine a negative feedback term 516, which can then be combined 518 with positive feedback 514 to generate a control signal for the motor 506.

Negative feedback may limit the positive feedback applied to the control signal to certain bounds, thereby suppressing the change to the control signal as the patient inhales or exhales. The negative feedback can include a minimum and/or maximum threshold flow rate value. A minimum flow rate value can ensure that the apparatus maintains the flow above a threshold, regardless of how hard the patient is breathing. This can ensure the effectiveness and/or safety of a high flow therapy. The maximum flow rate value can limit the amount of adjustment of the flow rate based on the patient's breath cycle even as the magnitude of the patient's inspiration or expiration increases.

The positive feedback 514 can include a breath synchronisation setting on the display of the apparatus adjustable by a user or care provider. The positive feedback breath synchronisation settings can allow the user or care provider to adjust the settings of the machine to further improve comfort and/or effectiveness by manually adjusting an amount of positive feedback. The settings can include, for example, any number of selectable values, such as 2-10, 2-5, 2, 3, 4, 5, or others. The selectable values can be labelled by being numbered. Alternatively, the selectable values can be labelled as different categories, such as high, medium, and/or low settings. Alternatively, the settings can allow the user to go higher (for example, by pressing a "+" button or the like) or lower (for example, by pressing a "-" button or the like) on the breath synchronization value.

The breath synchronisation can be introduced gradually, for example, by increasing the amplitude of the positive feedback while keeping the average target flow rate the same during the time when the amplitude is increased. The gradual introduction can occur when the breath synchronisation is first turned on, and/or when the user increases an expiratory relief level (that is, increasing the positive feedback level). The gradual introduction can cause the positive feedback to be slowly increased from zero or its previous level up to its new value over a predetermined duration, for example, about 30 seconds, about 1 minute, about 2 minutes, about 3 minutes, about 4 minutes or about 5 minutes. This can result in the motor speed oscillations, and in turn the flow rate oscillations, to slowly increase in amplitude. The gradual introduction can reduce potential patient discomfort during the initial stage of the breath synchronisation, in which the flow rate changes may not be fully synchronised with the patient's breathing. During the gradual introduction, the flow rate may begin to cross the flow rate thresholds. At this point, the negative feedback term can also begin to gradually increase, cancelling or substantially cancelling the further increase in the positive feedback term, in order to maintain the flow rate within the maximum and/or minimum thresholds.

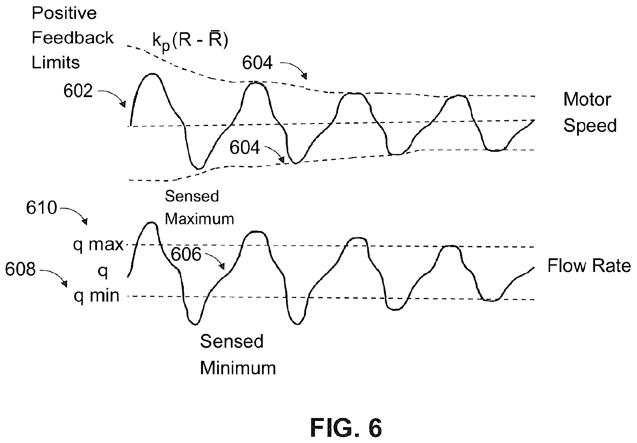

An example of the effect of positive feedback on the motor speed is illustrated in Figure 6. As shown in the top graph of Figure 6, the motor speed 602 varies over several breath periods. The motor speed can include a nominal motor speed and positive feedback, with the maximum and minimum values of the motor speed enveloped by the positive feedback term 604. There is a limit on the amount of positive feedback 604, which is initially set high enough so that no limiting occurs. Correspondingly, the initially sensed maximum and minimum flow rates are unbounded responses to the patient's breathing, and are gradually bounded by the negative feedback. The process for determining the flow rate, such as shown in Figure 5, does not know in advance the extent of adjustment of the motor speed so as to stay within the limit or threshold. The negative feedback therefore gradually bounds the positive feedback, for example, the negative feedback can be gradually increased until the measured or sensed maximum and/or minimum flow rates no longer exceeds the limit or threshold.

The minimum and/or maximum threshold flow rate can be fixed values, that is, regardless of the selected flow rate or the selected breath synchronization value. Alternatively or additionally, the minimum and/or maximum threshold flow rate can be set by the user. This can ensure that the device always delivers a minimum flow rate and/or never exceeds a maximum flow rate, regardless of the selected flow rate or the selected breath synchronisation value. Allowing the user to set the threshold(s) can also allow the flow therapy apparatus to deliver gases at a flow rate that is comfortable to the specific patient. The maximum and/or minimum thresholds can be set independent of the breath synchronization setting. For example, a clinician can be allowed to set a maximum and/or minimum threshold to ensure the effectiveness and/or safety of the high flow therapy, while the patient can be allowed to set the breath synchronization value based on what the patient find most comfortable.

Alternatively or additionally, the minimum and/or maximum flow rate can be determined based on a target flow rate set by the user. For example, the maximum and/or minimum flow rate can be a certain percentage above and below the selected flow rate. Alternatively or additionally, the maximum and/or minimum flow rate can be a certain value above and below the selected flow rate.

Alternatively or additionally, the minimum and/or maximum flow rate can be based on both the selected flow rate and the selected breath synchronization value. For example, the maximum and/or minimum flow rate can be a certain percentage above and below the selected flow rate. This percentage value can then vary based on the selected breath synchronization value. Alternatively, the maximum and/or minimum flow rate can be a certain value above and below the selected flow rate. This value can then vary based on the selected breath synchronization value.

The bottom graph of Figure 6 shows the flow rate 606 sensed by the flow rate sensor(s) of the apparatus and the minimum and maximum flow rates 608, 610 that have been set by the user. The flow rate 606 results from the motor speed changes according to the top graph of Figure 6 and the patient's positive feedback. To keep the flow rate within these limits 608, 610, the controller of the apparatus measures the minimum and maximum flow rate within each breath, and then iteratively adjusts the feedback loop to drive the flow within the bounds such as by following the process shown in Figure 5.

Example User Interfaces or Portions Thereof

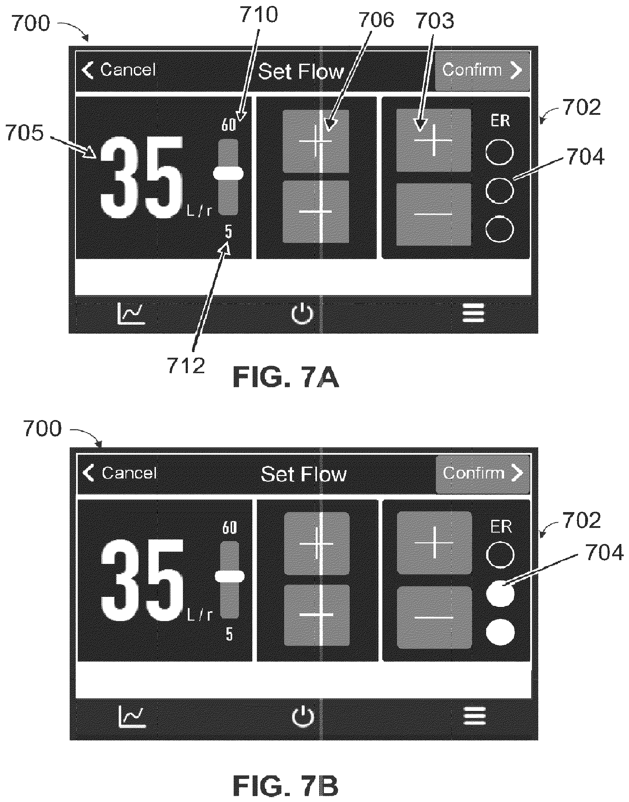

As shown in Figures 7A and 7B, the user interface 700, which can be the user interface 14 of Figure 1A, can display a setting that allows a user, such as a patient or a clinician, to adjust an expiratory relief ("ER") level 702. The expiratory relief levels correspond to the positive feedback terms or breath synchronization settings described above. As shown in Figures 7A and 7B, the user can choose four different levels of expiratory relief, which can include no expiratory relief (that is, no positive feedback) as illustrated by all three ER circles 704 being empty in Figure 7A, a first or lowest level of expiratory relief, a second level expiratory relief as illustrated by two of the three ER circles 704 being solid in Figure 7B, and a third or highest level of expiratory relief (that is, a highest or maximum positive feedback term). The user can adjust the expiratory relief level, for example, via buttons 703 or other forms of user input for increasing or decreasing the level.

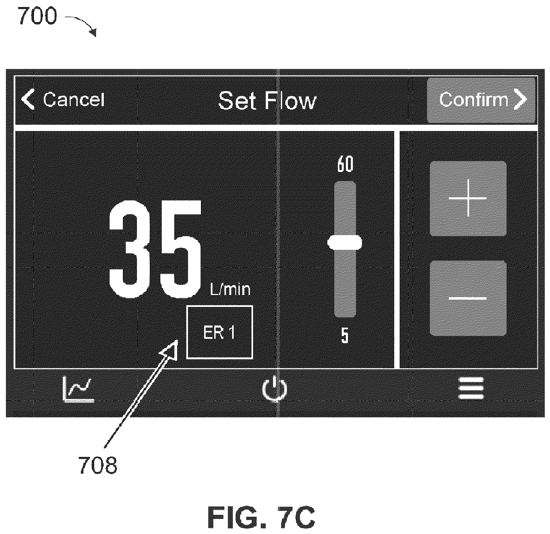

The ER level 702 as shown in Figures 7A and 7B illustrates an example expiratory relief setting. As described above, the user interface can display other forms of expiratory relief setting, such as numerical values, a sliding scale, or otherwise. The expiratory relief setting can include different numbers of levels, which can be discrete levels or on a continuum. As shown in Figure 7C, when the expiratory relief setting menu is collapsed, the user interface 700 can also display a previously selected expiratory relief level 708 on the screen (for example, below a target flow rate value). The previously selected expiratory relief level 708 can also optionally be displayed only when the user has set the amount of expiratory relief to a value above 0.

As shown in Figures 7A-7C, the user interface 700 can also optionally allow adjustments of a set flow rate 705, for example, via buttons 706 or other forms of user input for increasing or decreasing the set flow rate 705. The user interface 700 can also display a maximum 710 and minimum 712 flow rate threshold between which the user is allowed to adjust the set flow rate 705. In a configuration, the maximum and minimum flow rate thresholds can be set by a user (such as a clinician) on a separate menu. The separate menu may be designed to be used by the clinician, a technician and/or an engineer. The separate menu may be designed to be inaccessible to a patient or other regular users.

Flow Therapy System

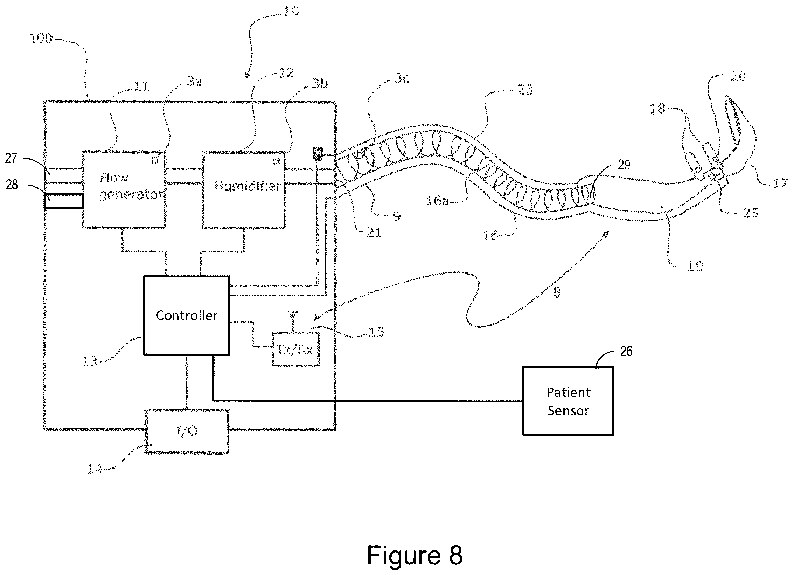

Referring additionally to Figure 8, a system 10 is shown that is similar to the system of Figure 1A. The system of Figure 8 additionally comprises an oxygen inlet port 28 for connection to a source of oxygen (such as a hospital oxygen supply or a oxygen canister or the like), in addition to ambient inlet port 27. The system of Figure 8 also comprises a temperature sensor 29 at or near the end of the inspiratory tube. The system 10 of Figure 8 is also suitable for implementing control of the flow of breathable gases in accordance with this disclosure.

Operation sensors 3a, 3b, 3c, such as flow, temperature, humidity, and/or pressure sensors can be placed in various locations in the flow therapy system 10. Additional sensors (for example, sensors 20, 25) may be placed in various locations on the patient conduit 16 and/or cannula 17 (for example, there may be a temperature sensor 29 at or near the end of the inspiratory tube). Output from the sensors can be received by the controller 13, to assist the controller in operating the flow therapy system 10 in a manner that provides suitable therapy. In some configurations, providing suitable therapy includes meeting a patient's peak inspiratory demand. The system 10 may have a transmitter and/or receiver 15 to enable the controller 13 to receive signals 8 from the sensors and/or to control the various components of the flow therapy system 10, including but not limited to the flow generator 11, humidifier 12, and heater wire 16a, or accessories or peripherals associated with the flow therapy system 10. Additionally, or alternatively, the transmitter and/or receiver 15 may deliver data to a remote server or enable remote control of the system 10.

Oxygen may be measured by placing one or more gas composition sensors (such as an ultrasonic transducer system, also referred to as an ultrasonic sensor system) after the oxygen and ambient air have finished mixing. The measurement can be taken within the device, the delivery conduit, the patient interface, or at any other suitable location.

Oxygen concentration may also be measured by using flow rate sensors on at least two of the ambient air inlet conduit, the oxygen inlet conduit, and the final delivery conduit to determine the flow rate of at least two gases. By determining the flow rate of both inlet gases or one inlet gas and one total flow rate, along with the assumed or measured oxygen concentrations of the inlet gases (about 20.9% for ambient air, about 100% for oxygen), the oxygen concentration of the final gas composition can be calculated. Alternatively, flow rate sensors can be placed at all three of the ambient air inlet conduit, the oxygen inlet conduit, and the final delivery conduit to allow for redundancy and testing that each sensor is working correctly by checking for consistency of readings. Other methods of measuring the oxygen concentration delivered by the flow therapy apparatus 10 can also be used.

The flow therapy system 10 can include a patient sensor 26, such as a pulse oximeter or a patient monitoring system, to measure one or more physiological parameters of the patient, such as a patient's blood oxygen saturation (SpO2), heart rate, respiratory rate, perfusion index, and provide a measure of signal quality. The sensor 26 can communicate with the controller 13 through a wired connection or by communication through a wireless transmitter on the sensor 26. The sensor 26 may be a disposable adhesive sensor designed to be connected to a patient's finger. The sensor 26 may be a non-disposable sensor. Sensors are available that are designed for different age groups and to be connected to different locations on the patient, which can be used with the flow therapy system 10. The pulse oximeter would be attached to the user, typically at their finger, although other places such as an earlobe are also an option. The pulse oximeter would be connected to a processor in the device and would constantly provide signals indicative of the patient's blood oxygen saturation. The patient sensor 26 can be a hot swappable device, which can be attached or interchanged during operation of the flow therapy system 10. For example, the patient sensor 26 may connect to the flow therapy system 10 using a USB interface or using wireless communication protocols (such as, for example, near field communication, WiFi or Bluetooth®). When the patient sensor 26 is disconnected during operation, the flow therapy system 10 may continue to operate in its previous state of operation for a defined time period. After the defined time period, the flow therapy system 10 may trigger an alarm, transition from automatic mode to manual mode, and/or exit control mode (e.g., automatic mode or manual mode) entirely. The patient sensor 26 may be a bedside monitoring system or other patient monitoring system that communicates with the flow therapy system 10 through a physical or wireless interface.

Control System

With reference again to Figure 8, the controller 13 can be programmed with or configured to execute a closed loop control system for controlling the operation of the flow therapy system 10. The closed loop control system can be configured to ensure the patient's SpO2 reaches a target level and consistently remains at or near this level.

The controller 13 can receive input(s) from a user that can be used by the controller 13 to execute the closed loop control system. The target SpO2 value can be a single value or a range of values. The value(s) could be pre-set, chosen by a clinician, or determined based on the type of patient, where type of patient could refer to current affliction, and/or information about the patient such as age, weight, height, gender, and other patient characteristics. Similarly, the target SpO2 could be two values, each selected in any way described above. The two values would represent a range of acceptable values for the patient's SpO2. The controller can target a value within said range. The targeted value could be the middle value of the range, or any other value within the range, which could be pre-set or selected by a user. Alternatively, the range could be automatically set based on the targeted value of SpO2. The controller can be configured to have one or more set responses when the patient's SpO2 value moves outside of the range. The responses may include alarming, changing to manual control of FdO2, changing the FdO2 to a specific value, and/or other responses. The controller can have one or more ranges, where one or more different responses occur as it moves outside of each range.

The graphical user interface of the flow therapy system 10 may be configured to prompt the user to input a patient type, and the SpO2 limits would be determined based on what the user selects. Additionally, the user interface may include a custom option, where the user can define the limits.

Generally, SpO2 would be controlled between about 80% and about 100%, or about 80% and about 90%, or about 88% and about 92%, or about 90% and about 99%, or about 92% and about 96%. The SpO2 could be controlled between any two suitable values from any two of the aforementioned ranges. The target SpO2 could be between about 80% and about 100%, or between about 80% and about 90%, or between about 88% and about 92%, or between about 90% and about 99%, or between about 92% and about 96%, or about 94%, or 94% or about 90%, or 90%, or about 85%, or 85%. The SpO2 target could be any value between any two suitable values from any two of the aforementioned ranges. The SpO2 target can correspond to the middle of the SpO2 for a defined range.

The FdO2 can be configured to be controlled within a range. As discussed previously, the oxygen concentration measured in the system (FdO2) would be substantially the same as the oxygen concentration the patient is breathing (FiO2) so long as the flow rate meets or exceeds the peak inspiratory demand of the patient, and as such the terms may can be seen as equivalent. Each of the limits of the range could be pre-set, selected by a user, or determined based on the type of patient, where the type of patient could refer to current affliction, and/or information about the patient such as age, weight, height, gender, and/or other patient characteristic. Alternatively, a single value for FdO2 could be selected, and the range could be determined at least partially based on this value. For example, the range could be a set amount above and below the selected FdO2. The selected FdO2 could be used as the starting point for the controller. The system could have one or more responses if the controller tries to move the FdO2 outside of the range. These responses could include alarming, preventing the FdO2 moving outside of the range, switching to manual control of FdO2, and/or switching to a specific FdO2. The device could have one or more ranges where one or more different responses occur as it reaches the limit of each range.

FdO2 can be controlled between about 21% and about 100%, or about 21% and about 90%, or about 21% and about 80%, or about 21% and about 70%, or about 21% and about 60%, or about 21% and about 50%, or about 25% and about 45%. The FdO2 could be controlled between any two suitable values from any two ranges described. The FdO2 target could be between any two suitable values from any two ranges described. If the range is based on the single value, the upper and lower limits could be decided by adding/subtracting a fixed amount from the selected value. The amount added or subtracted could be about 1%, or about 5%, or 10 %, or about 15%, or about 20%, or about 30%, or about 50%, or about 100%. The amount added/subtracted could change relative to the selected value. For example, the upper limit could be 20% higher than the selected value, so a selected value of 50% FdO2 would have an upper limit of 60% for the range of control. The percentage used for the range could be about 1%, or about 5%, or 10 %, or about 15%, or about 20%, or about 30%, or about 50%, or about 100%. The method for calculating the lower limit and the upper would not necessarily need to be the same. If a single value is used, the value could be between about 21% and about 100%, or about 25% and about 90%, or about 25% and about 80%, or about 25% and about 70%, or about 25% and about 60%, or about 25% and about 50%, or about 25% and about 45%.

Closed Loop Control

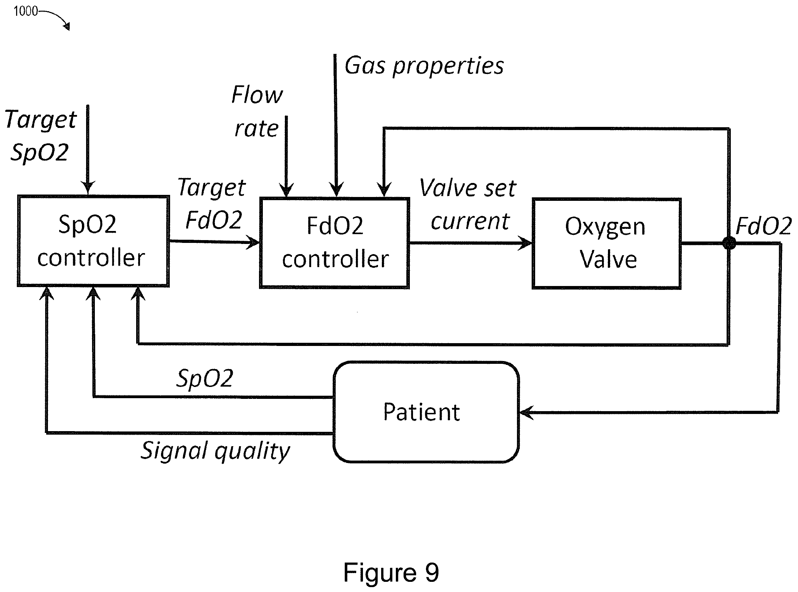

With reference to Figure 9 a schematic diagram of the closed loop control system 1000 is illustrated. The closed loop control system may utilize two control loops. The first control loop may be implemented by the SpO2 controller. The SpO2 controller can determine a target FdO2 based in part on the target SpO2 and/or the measured SpO2. As discussed above, the target SpO2 value can be a single value or a range of acceptable values. The value(s) could be pre-set, chosen by a clinician, or determined automatically based on client characteristics. Generally, target SpO2 values are received or determined before or at the beginning of a therapy session, though target SpO2 values may be received at any time during the therapy session. During a therapy session, the SpO2 controller can also receive as inputs: measured FdO2 reading(s) from a gases composition sensor, and measured SpO2 reading(s) and a signal quality reading(s) from the patient sensor. In some configurations, the SpO2 controller can receive target FdO2 as an input, in such a case, the output of the SpO2 controller may be provided directly back to the SpO2 controller as the input. Based at least in part on the inputs, the SpO2 controller can output a target FdO2 to the second control loop.

The second control loop may be implemented by the FdO2 controller. The FdO2 controller can receive inputs of measured FdO2 and target FdO2. The FdO2 controller can then output an oxygen inlet valve control signal to control the operation of the oxygen valve based on a difference between these measured FdO2 and target FdO2 values. The FdO2 controller may receive the target FdO2 value that is output from the first control loop when the flow therapy system 10 is operating in automatic mode. The FdO2 controller may also receive additional parameters such as flow rate values, gas properties, and/or measured FdO2. The gas properties may include the temperature of the gas at the O2 inlet and/or the oxygen content of the supply source. The gases supply source connected to the oxygen inlet valve may be an enriched oxygen gasflow where the oxygen content of the supply source may be less than pure oxygen (i.e., 100%). For example, the oxygen supply source may be an oxygen enriched gas flow having an oxygen content of less than 100% and greater than 21%.

From at least some of the inputs, the FdO2 controller can determine an oxygen flow rate that would be required to achieve the target FdO2. The FdO2 controller can use the flow rate input in order to alter the valve control signal. If the flow rate changes, the FdO2 controller can automatically calculate a new required oxygen flow rate required to maintain the target FdO2 at the new flow rate without having to wait for feedback from the gas concentration sensor, such as the measured FdO2 value. The FdO2 controller can then output the altered valve control signal to control the valve based on the new flow rate. In some configurations, the control signal of the FdO2 controller may set the current of the oxygen valve in order to control operation of the oxygen valve. Additionally, or alternatively, the FdO2 controller could detect changes to the measured FdO2 and alter the position of the valve accordingly. During manual mode, the second control loop can operate independently without receiving the target FdO2 from the first control loop. Rather, the target FdO2 can be received from user input or a default value.

During the therapy session, the SpO2 and FdO2 controllers can continue to automatically control the operation of the flow therapy system until the therapy session ends or an event triggers a change from the automatic mode to manual mode.

Terminology

Although this disclosure has been described in the context of certain embodiments and examples, it will be understood by those skilled in the art that the disclosure extends beyond the specifically disclosed embodiments to other alternative embodiments and/or uses and obvious modifications and equivalents thereof. In addition, while several variations of the embodiments of the disclosure have been shown and described in detail, other modifications, which are within the scope of this disclosure, will be readily apparent to those of skill in the art. It is also contemplated that various combinations or sub-combinations of the specific features and aspects of the embodiments may be made and still fall within the scope of the disclosure. For example, features described above in connection with one embodiment can be used with a different embodiment described herein and the combination still fall within the scope of the disclosure. It should be understood that various features and aspects of the disclosed embodiments can be combined with, or substituted for, one another in order to form varying modes of the embodiments of the disclosure. Thus, it is intended that the scope of the disclosure herein should not be limited by the particular embodiments described above.

Features, materials, characteristics, or groups described in conjunction with a particular aspect, embodiment, or example are to be understood to be applicable to any other aspect, embodiment or example described in this section or elsewhere in this specification unless incompatible therewith.

Furthermore, certain features that are described in this disclosure in the context of separate implementations can also be implemented in combination in a single implementation. Conversely, various features that are described in the context of a single implementation can also be implemented in multiple implementations separately or in any suitable subcombination. Moreover, although features may be described above as acting in certain combinations, one or more features from a claimed combination can, in some cases, be excised from the combination, and the combination may be claimed as a subcombination or variation of a subcombination.

Moreover, while operations may be depicted in the drawings or described in the specification in a particular order, such operations need not be performed in the particular order shown or in sequential order, or that all operations be performed, to achieve desirable results. Other operations that are not depicted or described can be incorporated in the example methods and processes. For example, one or more additional operations can be performed before, after, simultaneously, or between any of the described operations. Further, the operations may be rearranged or reordered in other implementations. Those skilled in the art will appreciate that in some embodiments, the actual steps taken in the processes illustrated and/or disclosed may differ from those shown in the figures. Depending on the embodiment, certain of the steps described above may be removed, others may be added. Furthermore, the features and attributes of the specific embodiments disclosed above may be combined in different ways to form additional embodiments, all of which fall within the scope of the present disclosure. Also, the separation of various system components in the implementations described above should not be understood as requiring such separation in all implementations, and it should be understood that the described components and systems can generally be integrated together in a single product or packaged into multiple products.

For purposes of this disclosure, certain aspects, advantages, and novel features are described herein. Not necessarily all such advantages may be achieved in accordance with any particular embodiment. Thus, for example, those skilled in the art will recognize that the disclosure may be embodied or carried out in a manner that achieves one advantage or a group of advantages as taught herein without necessarily achieving other advantages as may be taught or suggested herein.

Conditional language used herein, such as, among others, "can," "could," "might," "may," "e.g.," and the like, unless specifically stated otherwise, or otherwise understood within the context as used, is generally intended to convey that certain embodiments include, while other embodiments do not include, certain features, elements and/or steps. Thus, such conditional language is not generally intended to imply that features, elements and/or steps are in any way required for one or more embodiments or that one or more embodiments necessarily include logic for deciding, with or without other input or prompting, whether these features, elements and/or steps are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain embodiments, the terms "generally parallel" and "substantially parallel" refer to a value, amount, or characteristic that departs from exactly parallel by less than or equal to 15 degrees, 10 degrees, 5 degrees, 3 degrees, 1 degree, 0.1 degree, or otherwise.

Any methods disclosed herein need not be performed in the order recited. The methods disclosed herein include certain actions taken by a practitioner; however, they can also include any third-party instruction of those actions, either expressly or by implication. For example, actions such as "controlling a motor speed" include "instructing controlling of a motor speed."

All of the methods and tasks described herein may be performed and fully automated by a computer system. The computer system may, in some cases, include multiple distinct computers or computing devices (e.g., physical servers, workstations, storage arrays, cloud computing resources, etc.) that communicate and interoperate over a network to perform the described functions. Each such computing device typically includes a processor (or multiple processors) that executes program instructions or modules stored in a memory or other non-transitory computer-readable storage medium or device (e.g., solid state storage devices, disk drives, etc.). The various functions disclosed herein may be embodied in such program instructions, and/or may be implemented in application-specific circuitry (e.g., ASICs or FPGAs) of the computer system. Where the computer system includes multiple computing devices, these devices may, but need not, be co-located. The results of the disclosed methods and tasks may be persistently stored by transforming physical storage devices, such as solid state memory chips and/or magnetic disks, into a different state. In some embodiments, the computer system may be a cloud-based computing system whose processing resources are shared by multiple distinct business entities or other users.

The scope of the present invention is defined by the appended claims. The language of the claims is to be interpreted broadly based on the language employed in the claims and not limited to the examples described in the present specification or during the prosecution of the application, which examples are to be construed as non-exclusive.

**Claims**

1. A respiratory system (10) for delivering a respiratory therapy to a patient, the system configured to adjust a flow rate of gases delivered to the patient according to patient inspiration and expiration, the system (10) comprising:

   a flow generator (11) configured to generate the flow rate of gases; and
   a controller (13) in electrical communication with one or more sensors (3a, 3b, 3c, 20, 25) and configured to:

   determine a cycle of the patient inspiration and expiration based on information received from the one or more sensors (3a, 3b, 3c, 20, 25); and
   adjust the flow rate of gases based in part on the cycle of the patient inspiration and expiration, wherein the adjusting is attenuated by a parameter determined by comparing a maximum and/or minimum flow rate measured by the one or more sensors (3a, 3b, 3c, 20, 25) during the cycle of the patient inspiration and expiration with a maximum and/or minimum threshold;

   wherein the maximum and/or minimum flow rate measured by the one or more sensors (3a, 3b, 3c, 20, 25) is gradually bounded by a negative feedback term; and
   wherein the parameter comprises the negative feedback term **characterized in that** the negative feedback term is gradually increased in response to the measured maximum and/or minimum flow rates until the measured maximum and/or minimum flow rate no longer exceeds the maximum and/or minimum threshold.

2. The system (10) of Claim 1, wherein the controller (13) is configured to receive the maximum and/or minimum threshold set by a user.

3. The system (10) of Claim 2, wherein the system comprises a user interface (700) configured to receive a user input to adjust the maximum and/or minimum threshold.

4. The system (10) of any of Claims 1-3, wherein the maximum and/or minimum threshold is a clinically relevant limit of the respiratory therapy.

5. The system (10) of any of Claims 1-4, wherein the maximum and/or minimum threshold is a safety limit of the respiratory therapy.

6. The system (10) of any of Claims 1-5, wherein the controller (13) is configured to determine the maximum and/or minimum threshold based on a selected flow rate.

7. The system (10) of Claim 6, wherein the controller (13) is configured to determine the maximum and/or minimum threshold as a predetermined percentage or value above and below the selected flow rate.

8. The system (10) of Claim 6 or 7, wherein the controller (13) is configured to determine the maximum and/or minimum threshold based on a selected flow rate and a selected breath synchronization setting.

**Patentansprüche**

1. Beatmungssystem (10) zum Abgeben einer Beatmungstherapie an einen Patienten, wobei das System zum Anpassen einer Strömungsrate von an den Patienten gemäß Inspiration und Exspiration des Patienten abgegebenen Gasen konfiguriert ist, wobei das System (10) Folgendes umfasst:

einen Strömungsgenerator (11), der zum Erzeugen der Strömungsrate von Gasen konfiguriert ist; und
eine Steuerung (13) in elektrischer Verbindung mit einem oder mehreren Sensoren (3a, 3b, 3c, 20, 25) und konfiguriert zum:

Bestimmen eines Zyklus von Inspiration und Exspiration des Patienten basierend auf von dem einem oder den mehreren Sensoren (3a, 3b, 3c, 20, 25) empfangenen Informationen; und
Anpassen der Strömungsrate der Gase teilweise basierend auf dem Zyklus der Inspiration und Exspiration des Patienten, wobei das Anpassen durch einen Parameter verringert wird, der durch Vergleichen einer von dem einem oder den mehreren Sensoren (3a, 3b, 3c, 20, 25) während des Zyklus der Inspiration und Exspiration des Patienten gemessenen maximalen und/oder minimalen Strömungsrate mit einem maximalen und/oder minimalen Schwellenwert bestimmt wird;
wobei die durch den einen oder die mehreren Sensoren (3a, 3b, 3c, 20, 25) gemessene maximale und/oder minimale Strömungsrate allmählich durch einen Gegenkopplungsterm begrenzt wird; und
wobei der Parameter den Gegenkopplungsterm umfasst, **dadurch gekennzeichnet, dass** der Gegenkopplungsterm als Reaktion auf die gemessenen maximalen und/oder minimalen Strömungsraten allmählich erhöht wird, bis die gemessene maximale und/oder minimale Strömungsrate den maximalen und/oder minimalen Schwellenwert nicht länger überschreitet.

2. System (10) nach Anspruch 1, wobei die Steuerung (13) zum Empfangen des durch einen Benutzer eingestellten maximalen und/oder minimalen Schwellenwerts konfiguriert ist.

3. System (10) nach Anspruch 2, wobei das System eine Benutzeroberfläche (700) umfasst, die zum Empfangen einer Benutzereingabe zum Anpassen des maximalen und/oder minimalen Schwellenwerts konfiguriert ist.

4. System (10) nach einem der Ansprüche 1-3, wobei es sich bei dem maximalen und/oder minimalen Schwellenwert um einen klinisch relevanten Grenzwert der Beatmungstherapie handelt.

5. System (10) nach einem der Ansprüche 1-4, wobei es sich bei dem maximalen und/oder minimalen Schwellenwert um einen Sicherheitsgrenzwert der Beatmungstherapie handelt.

6. System (10) nach einem der Ansprüche 1-5, wobei die Steuerung (13) zum Bestimmen des maximalen und/oder minimalen Schwellenwerts basierend auf einer ausgewählten Strömungsrate konfiguriert ist.

7. System (10) nach Anspruch 6, wobei die Steuerung (13) zum Bestimmen des maximalen und/oder minimalen Schwellenwerts als einen vorbestimmten Prozentsatz oder Wert über und unter der ausgewählten Strömungsrate konfiguriert ist.

8. System (10) nach Anspruch 6 oder 7, wobei die Steuerung (13) zum Bestimmen des maximalen und/oder minimalen Schwellenwerts basierend auf einer ausgewählten Strömungsrate und einer ausgewählten Atemsynchronisierungseinstellung konfiguriert ist.

**Revendications**

1. Système respiratoire (10) destiné à administrer une thérapie respiratoire à un patient, le système étant configuré pour régler le débit du gaz administrés au patient en fonction de l'inspiration et de l'expiration du patient, le système (10) comprenant :

un générateur de flux (11) configuré pour générer le débit de gaz ; et

un dispositif de commande (13) en communication électrique avec un ou plusieurs capteurs (3a, 3b, 3c, 20, 25) et configuré pour :

déterminer un cycle de l'inspiration et de l'expiration du patient en fonction des informations reçues en provenance du ou des capteurs (3a, 3b, 3c, 20, 25) ; et

régler le débit des gaz en fonction en partie du cycle de l'inspiration et de l'expiration du patient, dans lequel le réglage est atténué par un paramètre déterminé en comparant un débit maximum et/ou minimum mesuré par le ou les capteurs (3a, 3b, 3c, 20, 25) pendant le cycle de l'inspiration et de l'expiration du patient avec un seuil maximum et/ou minimum ;

dans lequel le débit maximum et/ou minimum mesuré par le ou les capteurs (3a, 3b, 3c, 20, 25) est progressivement limité par un terme de rétroaction négatif ; et

dans lequel le paramètre comprend le terme de rétroaction négatif **caractérisé en ce que** le terme de rétroaction négatif est augmenté progressivement en réponse aux débits maximum et/ou minimum mesurés jusqu'à ce que le débit maximum et/ou minimum mesuré ne dépasse plus le seuil maximum et/ou minimum.

2. Système (10) selon la revendication 1, dans lequel le dispositif de commande (13) est configuré pour recevoir le seuil maximum et/ou minimum défini par un utilisateur.

3. Système (10) selon la revendication 2, dans lequel le système comprend une interface utilisateur (700) configurée pour recevoir une entrée utilisateur afin de régler le seuil maximum et/ou minimum.

4. Système (10) selon l'une quelconque des revendications 1 à 3, dans lequel le seuil maximum et/ou minimum est une limite cliniquement pertinente de la thérapie respiratoire.

5. Système (10) selon l'une quelconque des revendications 1 à 4, dans lequel le seuil maximum et/ou minimum est une limite de sécurité de la thérapie respiratoire.

6. Système (10) selon l'une quelconque des revendications 1 à 5, dans lequel le dispositif de commande (13) est configuré pour déterminer le seuil maximum et/ou minimum sur la base d'un débit sélectionné.

7. Système (10) selon la revendication 6, dans lequel le dispositif de commande (13) est configuré pour déterminer le seuil maximum et/ou minimum en tant que valeur ou pourcentage prédéterminé au-dessus et au-dessous du débit sélectionné.

8. Système (10) selon la revendication 6 ou 7, dans lequel le dispositif de commande (13) est configuré pour déterminer le seuil maximum et/ou minimum sur la base d'un débit sélectionné et d'un paramètre de synchronisation de respiration sélectionné.

**FIG. 1A**

EP 4 438 091 B1

**FIG. 1B**

**FIG. 1C**

200

201 → Temperature Sensor(s) Input

202 → Flow Rate Sensor(s) Inputs

203 → Motor Speed Inputs

204 → Pressure Sensor Input(s)

205 → Gas(es) Fraction Sensor Inputs

206 → Humidity Sensor(s) Inputs

207 → Pulse Oximeter Sensor(s) Input

208 → Stored or User Parameter(s)

209 → Duty/PWM Inputs

210 → Voltage(s) Inputs

211 → Current(s) Inputs

212 → Acoustic Sensor(s) Inputs

213 → Power(s) Inputs

214 → Resistance(s) Inputs

215 → CO2 Sensor(s) Inputs

216 → Spirometer Inputs

220 ↓

Control System

Blower Control Output(s) ← 230

Valve Control Output(s) ← 232

Heater Plate Control Output(s) ← 234

Heated Breathing Tube Control Output(s) ← 236

Audio Control Output(s) ← 238

Display Control Output(s) ← 239

# FIG. 2A

FIG. 2B

270

CONTROLLER

HARDWARE
PROCESSOR — 272

MEMORY — 274
CONTROL
SYSTEM — 276

CIRCUITS — 278

DISPLAY — 280

# FIG. 2C

FIG. 3A

FIG. 3B

Drive motor using control
signal
402

Flow rate
404a

Motor Speed
404b

Receive plurality of
measurements
404

Pressure
404c

Determine predicted breath
cycle based upon received
measurements
406

Adjust control signal based
upon predicted breath cycle
408

FIG. 4

FIG. 5

FIG. 6

EP 4 438 091 B1

FIG. 7A

FIG. 7B

700

‹ Cancel     Set Flow     Confirm ›

60

35 L/min

ER 1

5

708

**FIG. 7C**

Figure 8

Figure 9

**REFERENCES CITED IN THE DESCRIPTION**

**Patent documents cited in the description**

- WO 2017200394 A1
- NZ 2018050137 W
- WO 2019070136 A